# EUROPEAN PATENT APPLICATION

(11) **EP 4 400 107 A1**
(43) Date of publication of application: **17.07.2024**
(21) Application number: 22866772.1
(22) Date of filing: 09.09.2022
(51) Int. Cl.: A61K 31/50, A61K 9/127, C12N 15/88

(54) **IONIZABLE LIPOSOME, PREPARATION THEREOF, AND APPLICATION THEREOF IN GENE DELIVERY**

(30) Priority: 10.09.2021 CN 202111064429
(71) Applicant: Maxirna (Zhejiang) Technology Co., Ltd., Shaoxing, Zhejiang 312467 (CN); Maxirna (Shanghai) Pharmaceutical Co., Ltd., Songjiang District Shanghai 201601 (CN); Shanghai Cell Therapy Group Co., Ltd, Jiading District Anting Town Shanghai 201805 (CN)
(72) Inventor: TIAN, Zhidan, Shanghai 201805 (CN); XUE, Yuan, Shanghai 201805 (CN); LIU, Tao, Shanghai 201805 (CN); QIAN, Qijun, Shanghai 201805 (CN)
(74) Representative: van Dam, Vincent
(86) International application number: PCT/CN2022/118198
(87) International publication number: WO 2023/036311

(57) **Abstract**

Ionizable lipids, their preparation, and application in gene delivery. Specifically, provided are an ionizable lipid compound of the following formula I, and a stereoisomer, tautomer, pharmaceutically acceptable salt, prodrug, or solvate thereof, wherein the definition of each group in the formula is as stated herein. Further provided are lipid nanoparticles, comprising the ionizable lipid compound of formula (I). Further provided are a composition and a related use. The compound of formula (I) can be used for preparing the lipid nanoparticles for delivering nucleic acid-type therapeutic agents or active agents in vivo and in vitro.

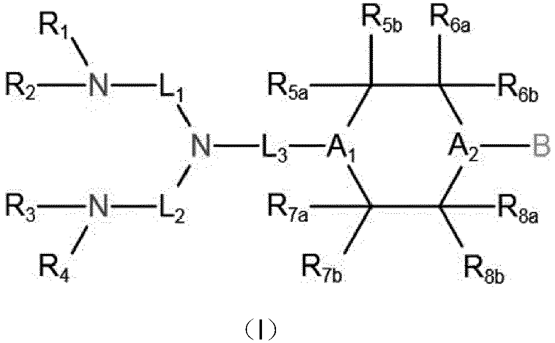

## Description

### Technical Field

The present description relates to ionizable lipids, their preparation and application in gene delivery.

### Technical Background

Nucleic acid drugs have become the third class of new drugs after small molecule drugs and antibody drugs. Different from other classes of drugs, nucleic acid drugs comprise nucleotide sequences. Currently, nucleic acid drugs having therapeutic effects include plasmids, messenger RNA (mRNA), antisense oligonucleotides (ASO), small interfering RNA (siRNA), microRNA (miRNA) and so on.

Nucleic acid and cell membrane both have negative charge, and it is difficult for naked nucleic acid to directly enter the cell without external force. At the same time, nucleic acid is easily degraded by nucleic acid degrading enzymes in the cytoplasm, therefore gene introduction and gene therapy cannot be realized. In order to achieve gene introduction, external force or carrier support is needed. Commonly used external force is acted through physical methods including electroporation, extrusion, injection and so on. The biggest disadvantage of these methods is that gene transfer in vivo cannot be performed, and gene transfer in vitro cannot be performed in large quantities.

Vectors are generally divided into viral vectors and non-viral vectors. Viral vectors have extremely high transfection efficiency in vivo and in vitro, but present many disadvantages, such as high toxicity, strong immune response, small gene load, poor targeting, and a complicated preparation process. On the other hand, non-viral vectors have the advantages of easy preparation, transportation, and storage, and are safe, effective, and non-immunogenic, which have drawn more and more attention and been applied widely.

Lipid nanoparticle (LNP) technology is a non-viral vector technology developed in recent years. Using this technology, Alnylam launched the world's first siRNA drug, Onpattro^{®}. Pfizer, Moderna and other companies have developed mRNA COVID-19 vaccines. Lipid nanoparticles (LNP) are prepared from ionizable cationic lipids and auxiliary lipids (phospholipids, cholesterol, and PEGylated lipids) through microfluidic equipment, and the auxiliary lipids have been commercialized. Ionizable cationic lipids directly determine the encapsulation and delivery efficiency of nucleic acid drugs and become the core element of LNP technology development.

T cell (T lymphocyte) is a kind of lymphocyte and plays an important role in immune response. With the development of cellular immunotherapy, especially the application of CAR-T technology, the genetic engineering of T cells is becoming more and more important. However, T cell transfection is still dependent on electroporation or viral vectors. Research on LNP or liposome-transfected T cells is very limited.

### Summary of the Description

In order to overcome the deficiencies of the prior art, the present description provides a delivery vector that can be used to deliver genes to cells, a preparation method and application thereof.

The first aspect of the present description provides an ionizable lipid compound of formula I, or stereoisomer, tautomer, pharmaceutically acceptable salt, prodrug or solvate thereof: 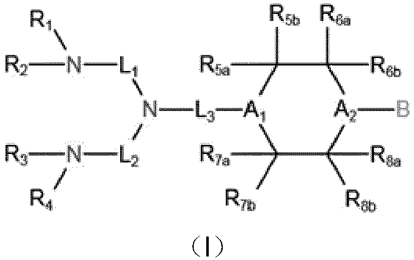 wherein,
A₁ and A₂ are each independently CH or N;
B is selected from -L₄-N (R₉R₁₀) or R₁₂;
L₁, L₂, L₃ and L₄ are each independently selected from optionally substituted alkylene, optionally substituted -[(CH₂)ₙO]ₘ-(CH2)ₒ-, optionally substituted alkenylene and optionally substituted alkynylene;
R₁, R₂, R₃, R₄, R₉, R₁₀ and R₁₂ are each independently H, halogen, optionally substituted alkyl, optionally substituted alkenyl, optionally substituted cycloalkyl, optionally substituted heterocyclyl, optionally substituted cycloalkenyl, optionally substituted cycloalkynyl, optionally substituted aryl, optionally substituted heteroaryl, optionally substituted alkoxy, optionally substituted alkoxycarbonyl, optionally substituted acyl, -NR'R", carboxy, nitro, cyano, or alkylsulfoxide;
R₅ₐ, R_{5b}, R₆ₐ, R_{6b}, R₇ₐ, R_{7b}, R₈ₐ and R_{8b} are each independently selected from H, optionally substituted alkyl, optionally substituted alkenyl, optionally substituted alkynyl, halogen, nitro, cyano, -NR'R" and carboxy;
n, m, and o are each independently an integer from 1 to 10; and
R' and R" are each independently selected from H or optionally substituted alkyl.

The second aspect of the present description provides a lipid nanoparticle comprising an ionizable lipid compound of formula I.

The third aspect of the present description provides a composition, which comprises the ionizable lipid compound of formula I, or comprises the lipid nanoparticle.

The fourth aspect of the present description provides a method of treating or preventing a disease or condition in a subject, the method comprising administering the pharmaceutical composition according to any embodiment of the present description.

The fifth aspect of the present description provides use of the compound of formula I described in any embodiment of the present description in manufacture of lipid nanoparticles or pharmaceutical compositions.

### Description of Figures

Figure 1: Cell viability analyzed by Flow cytometer.
Figure 2: The transfection efficiency of the LNP preparation according to one embodiment of the present description to cells under the condition of no addition or addition of AopE4.
Figure 3: Fluorescence imaging generated by cell fluorescence imaging.
Figure 4: Fluorescent imaging of mice after intramuscular (i.m) and intravenous (i.v) injection of lipid nanoparticles for 6 h.
Figure 5: Histological fluorescent imaging of mice after intramuscular (i.m) and intravenous (i.v) injection of lipid nanoparticles for 6 h.
Figure 6: Fluorescent imaging of mice after intramuscular (i.m) and intravenous (i.v) injection of lipid nanoparticles for 24 h.
Figure 7: Fluorescent intensity of mice after intramuscular (i.m) and intravenous (i.v) injection of lipid nanoparticles for 6 h, 24h, respectively.
Figure 8: Results of in vitro stability experiments.

### Detailed Description

It should be understood that within the scope of the present description, the above-mentioned technical features of the present description and the technical features specifically described in the following (such as the examples) can be combined with each other to form a preferred technical solution.

### I. Terminology

Unless otherwise indicated, the following terms used herein have the following meanings:
As used herein, "comprises", "includes" and "contains" and the like are to be construed in an open and inclusive sense, and throughout the specification and claims it means "including but not limited to" unless the context requires otherwise.

In the present description, reference to "one embodiment" means that a particular feature, structure, or characteristic described in connection with the embodiment is included in at least one embodiment of the present description. Thus, appearances of the phrase "in one or more embodiments" in various places throughout this specification are not necessarily all referring to the same embodiment. Furthermore, the particular features, structures or characteristics may be combined in any suitable manner in one or more embodiments.

Unless defined otherwise, all technical and scientific terms used herein have the same meaning as commonly understood by one of ordinary skill in the art to which this description belongs. As used in the specification and claims, the singular forms "a", "said" and "the" include plural referents unless the context clearly dictates otherwise.

An "effective amount" or "therapeutically effective amount" of an active agent or therapeutic agent, such as a therapeutic nucleic acid, is sufficient amount to produce a desired effect (such as an increase or inhibition of expression of a target sequence compared to normal expression levels detected in the absence of the nucleic acid).

As used herein, "nucleic acid" refers to a polymer comprising at least two deoxyribonucleotides or ribonucleotides in single- or double-stranded form, including DNA, RNA, and hybrids thereof. The DNA can be in the form of an antisense molecule, plasmid DNA, cDNA, PCR product, or vector. RNA can be in the form of small hairpin RNA (shRNA), messenger RNA (mRNA), antisense RNA, small interfering RNA (siRNA), microRNA (miRNA), multivalent RNA, Dicer substrate RNA, or viral RNA (vRNA) and combination thereof.

A "gene" as used herein refers to a nucleic acid sequence (such as DNA or RNA) that includes a partial or entire length coding sequence necessary to produce a polypeptide or precursor polypeptide.

"Lipids", as used herein, refer to a group of organic compounds that include, but are not limited to, esters of fatty acids, and are generally characterized as being poorly soluble in water but soluble in many organic solvents. They are generally divided into at least three categories: (1) "simple lipids", which include fats and oils as well as waxes; (2) "complex lipids", which include phospholipids and glycolipids; and (3) "derived lipids", which like steroids.

"Steroids" are compounds that contain the following carbon skeleton:

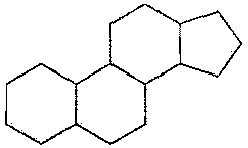

Non-limiting examples of steroids include cholesterol and the like.

"Cholesterol derivatives" may be cholesterol derivatives known in the art for preparing liposomes, and exemplary cholesterol derivatives include commonly used cholesterol, CAS: 57-88-5.

A "polymer-conjugated lipid" as used herein refers to a molecule comprising a lipid moiety and a polymer moiety. Examples of polymer-conjugated lipids are PEGylated (PEGed) lipids. The term "PEGylated lipid" refers to a molecule comprising a lipid moiety and a polyethylene glycol moiety. PEGylated lipids are known in the art and include 1-(monomethoxy-polyethylene glycol)-2,3-dimyristoylglycerol (PEG-DMG), PEG-DAG (diacylglycerol), PEG-PE (phosphatidylethanolamine), PEG-S-DAG, PEG-DSPE (-distearoylphosphatidylethanolamine), PEG-cer (ceramide) and PEG dialkoxypropyl carbamate, etc. In a preferred embodiment of the description, the polymer-conjugated lipid is mPEG₂₀₀₀-DSPE.

As used herein, "neutral lipid" refers to a lipid substance that exists in an uncharged or neutral zwitterionic form at a selected pH. At physiological pH, the lipids include, but are not limited to: phosphatidylcholines such as 1,2-distearoyl-sn-glyceryl-3-phosphocholine (DSPC), 1,2-dipalmitoyl-sn-glyceryl-3-phosphocholine (DPPC), 1,2-dimyristoyl-sn-glyceryl-3-phosphocholine (DMPC), 1-palmitoyl-2-oleoyl-sn-g lyceryl-3-phosphocholine (POPC), 1,2-dioleoyl-sn-glyceryl-3-phosphocholine (DOPC), phosphatidylethanolamine such as 1,2-dioleoyl-sn-glyceryl-3-phosphoethanolamine (DOPE), sphingomyelin (SM) and ceramide. Neutral lipids can be of synthetic or natural origin.

As used herein, "lipid nanoparticles" refer to particles having at least one nanometer-scale size (eg, 1-1000 nm). Lipid nanoparticles can be included in formulations for delivering active agents or therapeutic agents (eg, nucleic acids) to target sites of interest (eg, cells, tissues (eg, diseased tissues such as tumor tissue), organs). In some embodiments, lipid nanoparticles of the description comprise nucleic acids. The lipid nanoparticles generally comprise one or more compounds of formula I according to the description, one or more auxiliary lipid molecules, one or more cholesterol or cholesterol derivatives and/or one or more polymer-conjugated lipid molecules. The auxiliary lipid molecule can be one or more neutral lipid molecules. The active agent or therapeutic agent can be encapsulated in the lipid portion of the lipid nanoparticle or in an aqueous space encapsulated by some or all of the lipid portion of the lipid nanoparticle, thereby protecting it from enzymatic degradation, or from other undesired effects induced by the host organism's or cell's mechanisms, such as adverse immune responses.

As is known in the art, lipid nanoparticles can have an average diameter of about 30 nm to about 150 nm, about 40 nm to about 150 nm, about 50 nm to about 150 nm, about 60 nm to about 130 nm, about 70 nm to about 110 nm, about 70 nm to about 100 nm, about 80nm to about 100nm, about 90nm to about 100nm, about 70nm to about 90nm, about 80nm to about 90nm, about 70nm to about 80nm, or about 30nm, about 35nm, about 40nm, about 45nm, about 50nm, about 55nm, about 60nm , about 65nm, about 70nm, about 75nm, about 80nm, about 85nm, about 90nm, about 95nm, about 100nm, about 105nm, about 110nm, about 115nm, about 120nm, about 125nm, about 130nm, about 135nm, about 140nm, about 145 nm or about 150 nm, and the lipid nanoparticles are essentially non-toxic. In certain embodiments, the nucleic acid, when present in the lipid nanoparticle, is resistant to degradation by nucleases in aqueous solution. Lipid nanoparticles containing nucleic acids and their preparation methods are known in the prior art, for example, see CN102712935A or related patents, etc., the entire disclosure content of which is incorporated herein by reference in its entirety for all purposes.

The form of the lipid nanoparticle of the present description is not particularly limited, but examples of forms in which the ionizable lipid of the present description is dispersed in an aqueous solvent include unilamellar liposomes, multilamellar liposomes, or unspecified layered structures, etc.

Herein, "halogen" means fluorine, chlorine, bromine or iodine.
"Hydroxyl" means an -OH group.
"Carbonyl" means an -C(=O)- group.
"Carboxy" refers to -COOH.
"Nitro" refers to -NO₂.
"Cyano" refers to -CN.
"Amino" refers to -NH₂.

"Alkyl" refers to a straight-chain or branched saturated aliphatic hydrocarbon group containing from one to twenty-four carbon atoms, such as methyl, ethyl, propyl, butyl, tridecyl, heneicosyl, triacontanyl, methylpentadecyl, hexylnonyl, etc., and the alkyl is attached to the rest of the molecule by a single bond. In some embodiments, the alkyl groups described herein have 10-24 carbon atoms, alternatively have 12-20 carbon atoms.

"Alkylene" is a saturated, branched or straight chain hydrocarbon group having from 1 to 20 carbon atoms and having two monovalent radical centers obtained by removal of two hydrogen atoms from the same or two different carbon atoms of the parent alkane, such as -CH₂-, -CH₂CH₂-, -CH₂CH₂CH₂-, -CH₂CH₂CH₂CH₂- and -CH₂CH(CH₃)CH₂-, etc. In some embodiments, the alkylene described herein have 1-10 carbon atoms, or, for example, 1-4 carbon atoms.

"Alkoxy" refers to alkyl-O-, that is, an alkyl containing one to twenty-four carbon atoms with an oxygen atom attached to the end, such as methoxy, ethoxy, propyloxy, isopropoxy etc. Alkyleneoxy refers to an alkylene with O attached to one end, such as -CH₂CH₂-O-.

"Alkoxycarbonyl" refers to a group with a carbonyl group attached to the oxygen of a alkoxy, such as CH₃OC(=O)-, CH₃CH₂OC(=O)- and the like.

"Alkylsulfinyl" means an alkyl-S(=O)- group.

"Alkenyl" or "chain alkenyl" means a straight or branched chain aliphatic hydrocarbon group containing two to twenty-four carbon atoms, which contains one or more unsaturated carbon-carbon double bonds, such as vinyl, propenyl, tridecene group, tetradecadienyl, octadecatrienyl, etc., and is attached to the rest of the molecule by a single bond.

"Alkenylene" means a divalent straight-chain or branched alkenyl, usually having two to twenty-four carbon atoms, containing one or more unsaturated carbon-carbon double bonds and connected to the rest of the molecule through two single bonds. An exemplary alkenylene can be -CHCH=CHCH-.

"Cycloalkenyl" means an unsaturated cyclic alkenyl group containing three to ten ring carbon atoms.

"Alkynyl" refers to a straight-chain or branched aliphatic hydrocarbon group containing two to twenty-four carbon atoms, which contains one or more unsaturated carbon-carbon triple bonds, such as ethynyl, propynyl, etc., and connected to the rest of the molecule through a single bond.

"Cycloalkynyl" refers to an unsaturated cyclic alkenyl containing three to ten ring carbon atoms.

"Alkynylene" refers to a divalent straight-chain or branched alkynyl, usually having two to twenty-four carbon atoms, containing one or more unsaturated carbon-carbon triple bonds and connected to the rest of the molecule through two single bonds. An exemplary alkenylene can be -CHC≡CCH-.

"Acyl" refers to an alkyl-C(O)- group such as acetyl, propionyl, and the like.

"Aryl" refers to a carbocyclic ring system group comprising hydrogen, 6 to 18 carbon atoms (preferably 6-14 carbon atoms), and at least one aromatic ring. Aryl can be monocyclic, bicyclic, tricyclic or tetracyclic ring systems, which can include fused or bridged ring systems. Aryl include, but are not limited to, aryl groups of derived from aceanthrylene, acenaphthylene, acephenanthrylene, anthracene, azulene, benzene, fluoranthene, fluorene, asymmetric indacene, symmetric indacene, indane, indene, naphthalene, phenacene, phenanthrene, heptene, pyrene, and triphenylene.

"Aryloxy" means -OR, where R is aryl.

"Carbocyclyl" refers to a stable saturated or unsaturated non-aromatic monocyclic or polycyclic hydrocarbon group composed only of carbon and hydrogen atoms, and the number of ring carbon atoms may be 3-18. Carbocyclyl can include fused or bridged ring systems having 3 to 18 carbon atoms, are saturated and are attached to the rest of the molecule by a single bond. In some embodiments, the carbocyclyl is a cycloalkyl, representative cycloalkyls include, but are not limited to, those having three to fifteen carbon atoms (C₃-C₁₅ cycloalkyl), three to ten carbon atoms (C₃-C₁₀ cycloalkyl), three to eight carbon atoms (C₃-C₈ cycloalkyl), three to six carbon atoms (C₃-C₆ cycloalkyl), three to five carbon atoms (C₃-C₅ cycloalkyl) or a cycloalkyl with three to four carbon atoms (C₃-C₄ cycloalkyl). Monocyclic cycloalkyl include, for example, cyclopropyl, cyclobutyl, cyclopentyl, cyclohexyl, cycloheptyl and cyclooctyl. Polycyclic cycloalkyls include, for example, adamantyl, norbornyl, decalinyl, bicyclo[3.3.0]octane, bicyclo[4.3.0]nonane, cis-decalin, trans-decalin, bicyclo[2.1.1]hexane, bicyclo[2.2.1]heptane, bicyclo[2.2.2]octane, bicyclo[3.2.2]nonane and bicyclo[3.3.2]decane and 7,7- dimethyl-bicyclo[2.2.1]heptanyl. In a preferred embodiment, the cycloalkyl is a 3-8 membered cycloalkyl, such as cyclopropyl, cyclopentyl and cyclohexyl, etc.

"Heterocyclyl" refers to a stable 3-membered to 20-membered non-aromatic cyclic group consisting of 2 to 14 carbon atoms and 1 to 6 heteroatoms selected from nitrogen, phosphorus, oxygen and sulfur. The heterocyclyl can be a monocyclic, bicyclic, tricyclic or multicyclic ring system, which can include fused, bridged or spirocyclic systems. A heterocyclyl can be partially or fully saturated. A heterocyclyl can be attached to the rest of the compound via a carbon atom or a heteroatom and by a single bond. The heterocyclyl is preferably a stable 4-membered to 11-membered non-aromatic monocyclic, bicyclic, bridged or spirocyclic group containing 1 to 3 heteroatoms selected from nitrogen, oxygen and sulfur, more preferably a stable 4-membered to 8-membered non-aromatic monocyclic, bicyclic, bridged or spirocyclic group containing 1 to 3 heteroatoms selected from nitrogen, oxygen and sulfur. Examples of heterocyclyls include, but are not limited to: pyrrolidinyl, morpholinyl, piperazinyl, homopiperazinyl, piperidinyl, thiomorpholinyl, 2,7-diaza-spiro[3.5]nonane-7-yl, 2-oxa-6-aza-spiro[3.3]heptane-6-yl, 2,5-diaza-bicyclo[2.2.1]heptane-2-yl, aza cyclobutanyl, pyranyl, tetrahydropyranyl, thiopyranyl, tetrahydrofuryl, oxazinyl, dioxolyl, tetrahydroisoquinolyl, decahydroisoquinolyl, imidazolinyl, imidazolidinyl, quinazinyl, thiazolidinyl, isothiazolidinyl, isoxazolidinyl, indolinyl, octahydroindolyl, octahydroisoindolyl, pyrrolidinyl, pyrazolidinyl , phthalimide, etc.

"Heteroaryl" refers to a 5-membered to 16-membered conjugated ring system having 1 to 15 carbon atoms (preferably 1 to 10 carbon atoms) and 1 to 6 heteroatoms selected from nitrogen, oxygen and sulfur. Heteroaryls can be monocyclic, bicyclic, tricyclic or multicyclic ring systems. Heteroaryl is preferably a stable 5-membered to 12-membered aromatic group comprising 1 to 5 heteroatoms selected from nitrogen, oxygen and sulfur, more preferably a stable 5-membered to 10-membered aromatic groups comprising 1 to 4 heteroatoms selected from nitrogen, oxygen and sulfur, or 5-membered to 6-membered aromatic groups containing 1 to 3 heteroatoms selected from nitrogen, oxygen and sulfur. Examples of heteroaryls include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furyl, pyrrolyl, triazolyl, tetrazolyl, triazinyl, indolyl, isoindolyl, indazolyl, isoindazolyl , purinyl, quinolinyl, isoquinolinyl, diazinyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, phenylthio, indolizyl, o-phenanthrenyl, isoxazolyl, phenoxazinyl, phenothiazinyl, 4,5,6,7-tetrahydrobenzo[b]thienyl, naphthopyridyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazo[1,2-a]pyridine, imidazo[1,2-b]pyridazine, imidazo[1,2-a]pyrazine, etc.

Herein, when a group is "optionally substituted", it may be optionally substituted with 1-5 substituents selected from: alkyl, alkenyl, alkynyl, halogen, haloalkyl, haloalkenyl, haloalkynyl, cyano, nitro, optionally substituted aryl, optionally substituted heteroaryl, optionally substituted cycloalkyl, optionally substituted heterocyclyl. Each of these aryl, heteroaryl, cycloalkyl and heterocyclic groups as substituents may optionally substituted by groups such as alkyl, alkenyl, alkynyl, halogen, haloalkyl, haloalkenyl, haloalkynyl, cyano, nitro, aryl, heteroaryl, cycloalkyl and heterocyclyl. It is understood that the number of substituents is influenced by the molecular structure of the compound. For example, when the substituent is aryl, heteroaryl, cycloalkyl or heterocyclyl, the number of substituents is usually one; when the substituent is halogen, the number of halogen atoms can be 2-5, according to the chain length of the substituted group or the number of atoms of ring carbon.

Herein, "ionizable lipid compound" refers to a lipid compound that exists in a charged form within a specific pH value or pH range, including positively charged or negatively charged, preferably positively charged lipid compounds (i.e, cationic lipids compound). The specific pH value or pH range refers to the pH value or pH range of the environment in which the lipid compound is stored or intended to be used, including but not limited to physiological pH.

Herein, "pharmaceutically acceptable salts" include acid addition salts and base addition salts.

"Pharmaceutically acceptable acid addition salt" refers to a salt formed with an inorganic acid or an organic acid while retaining the biological efficacy and properties of the free base. The inorganic acid includes hydrochloric acid, hydrobromic acid, sulfuric acid, nitric acid, phosphoric acid and the like. The organic acids include acetic acid, 2,2-dichloroacetic acid, adipic acid, alginic acid, ascorbic acid, aspartic acid, benzenesulfonic acid, benzoic acid, 4-acetylaminobenzoic acid, camphoric acid, camphor-10-sulfonic acid, capric acid, caproic acid, caprylic acid, carbonic acid, cinnamic acid, citric acid, cyclamate, lauryl sulfate, ethane-1,2-disulfonic acid, ethanesulfonic acid, 2-hydroxyethanesulfonic acid , formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid , hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxyl-2-naphthoic acid, niacin, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid and undecylenic acid, etc.

"Pharmaceutically acceptable base addition salts" refer to those salts that retain the biological effectiveness and properties of the free acids. These salts are prepared by adding an inorganic or organic base to the free acid. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, potassium salts, calcium salts and magnesium salts. Salts derived from organic bases include, but are not limited to, salts of primary, secondary, and tertiary amines, substituted amines, including naturally occurring substituted amines, cyclic amines, and basic ion exchange resins such as ammonia, isopropylamine, trimethylamine, diethylamine, triethylamine, tripropylamine, diethanolamine, ethanolamine, diano, 2-dimethylaminoethanol, 2-diethylaminoethanol, dicyclohexylamine, lysine, arginine, histidine, caffeine, procaine, hybamine, choline, betaine, benamine, benzathine, ethylenediamine, glucosamine, methylglucosamine, theobromine, triethanolamine, tromethamine, purine, piperazine, piperidine, N-ethylpiperidine, polyamine resin, etc. Particularly preferred organic bases are isopropylamine, diethylamine, ethanolamine, trimethylamine, dicyclohexylamine, choline and caffeine.

Herein, "pharmaceutical composition" refers to a preparation containing the compound of formula I of the present invention and a pharmaceutically acceptable carrier or excipient. Pharmaceutical compositions generally contain a therapeutically effective amount of a compound of formula I and/or a therapeutic agent or active agent. A "therapeutically effective amount" refers to an amount sufficient to effect treatment in a mammal when administered to a mammal, preferably a human. The amount of a compound of formula I and/or therapeutic agent or active agent of the present description which constitutes a "therapeutically effective amount" will vary depending on the compound or therapeutic agent or active agent used, the condition and its severity, the route of administration and the age of the mammal to be treated, but can be routinely determined by one of ordinary skill in the art based on their knowledge and the present disclosure.

The "pharmaceutically acceptable carrier or excipient" described herein includes, but is not limited to, any adjuvant, carrier, excipient, glidant, sweetener, diluent, preservative, dye/colorant, flavor enhancer, surfactant, wetting agent, dispersant, suspending agent, stabilizer, isotonic agent, solvent or emulsifier that has been approved by FDA or CFDA for use in humans or domesticated animals.

"Treatment" as described herein encompasses treatment for the disease or condition of interest in a mammal, preferably a human, suffering from the disease or condition of interest, and includes:
(1) preventing the disease or condition from occurring in a mammal, especially when such mammal is susceptible to the condition but has not been diagnosed as having the condition;
(2) inhibiting the disease or condition, that is, prevent its development;
(3) amelioration of the disease or condition, that is, causing regression of the disease or condition; or
(4) relief of symptoms caused by the disease or condition, i.e., pain relief without curing the underlying disease or condition.

Herein, "disease" and "condition" may be used interchangeably, or may be different, because a particular disease or condition may not have a known causative agent (so that the cause has not yet been identified), and therefore it has not been recognized as a disease and is only considered as an undesired condition or syndrome in which a more or less specific group of symptoms has been identified by a clinician.

Herein, "mammal" includes humans as well as domestic animals such as laboratory animals and household pets (eg cats, dogs, pigs, cows, sheep, goats, horses, rabbits) and non-domestic animals (eg wild animals, etc.).

Compounds of formula I, or pharmaceutically acceptable salts thereof, may contain one or more asymmetric centers and thus give rise to enantiomers, diastereomers and other stereoisomeric forms defined as (R)- or (S)- or (D)- or (L)- (for amino acids) in terms of absolute stereochemistry. The present description includes all such possible isomers, as well as their racemic and optically pure forms. Optically active (+) and (-), (R)- and (S)-, or (D)- and (L)-isomers can be prepared using chiral synthons or chiral reagents, or by resolution using conventional techniques such as chromatography and fractional crystallization. Conventional techniques for the preparation/isolation of individual enantiomers include chiral synthesis from suitable optically pure precursors or resolution of racemates (or salts or derivatives) by, for example, chiral high pressure liquid chromatography (HPLC). When the compounds described herein contain olefinic double bonds or other geometric asymmetric centers, and unless otherwise stated, it is intended that the compounds include both E and Z geometric isomers. Likewise, all tautomeric forms are also intended to be included.

### II. Ionizable Lipid Compounds

The ionizable lipid compound described herein has the structural formula shown in the following formula I: 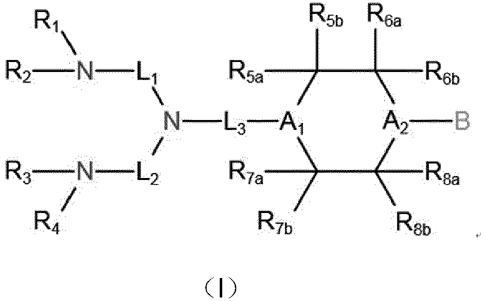 Wherein,
A₁ and A₂ are each independently CH or N; B is selected from -L₄-N (R₉R₁₀) or R₁₂; L₁, L₂, L₃ and L₄ are each independently selected from optionally substituted alkylene, optionally substituted -[(CH₂)ₙO]ₘ-(CH2)ₒ-, optionally substituted alkenylene and optionally substituted alkynylene; R₁, R₂, R₃, R₄, R₉, R₁₀ and R₁₂ are each independently H, halogen, optionally substituted alkyl, optionally substituted alkenyl, optionally substituted cycloalkyl, optionally substituted heterocyclyl, optionally substituted cycloalkenyl, optionally substituted cycloalkynyl, optionally substituted aryl, optionally substituted heteroaryl, optionally substituted alkoxy, optionally substituted alkoxycarbonyl, optionally substituted acyl, -NR'R", carboxy, nitro, cyano, or alkylsulfoxide; R₅ₐ, R_{5b}, R₆ₐ, R_{6b}, R₇ₐ, R_{7b}, R₈ₐ and R_{8b} are each independently selected from H, optionally substituted alkyl, optionally substituted alkenyl, optionally substituted alkynyl, halogen, nitro, cyano, -NR'R" and carboxyl; n, m, and o are each independently an integer from 1 to 10; and R' and R" are each independently selected from H or optionally substituted alkyl.

Stereoisomers, tautomers, pharmaceutically acceptable salts, prodrugs and solvates of the compounds of formula I are also included herein.

In formula I, when a group is "optionally substituted", its substituents can be selected from hydroxyl, alkoxy, halogen, alkyl, haloalkyl, hydroxyl-substituted alkyl, alkenyl, haloalkenyl, hydroxyl-substituted alkenyl, -NR'R", optionally substituted cycloalkyl, optionally substituted aryl, optionally substituted heteroaryl, or optionally substituted heterocyclyl. The number of substituents can be 1, 2, 3, 4, 5 or 6. The R' and R" are each independently selected from H and C₁₋₄ alkyl. The alkyl of alkyl, alkoxy, haloalkyl and hydroxyl-substituted alkyl can be C₁₋₆ alkyl or C₁₋₄ alkyl; the alkenyl of alkenyl, haloalkenyl, hydroxyl-substituted alkenyl can be C₂₋₆ alkenyl or C₂₋₄ alkenyl; the cycloalkyl can be C₃₋₈ cycloalkyl, optionally substituted by 1-4 substituents selected from halogen, C₁₋₄ alkyl, hydroxyl and -NR'R"; the aryl can be 6-14 membered aryl, such as phenyl and naphthyl, the aryl can be optionally substituted by 1-4 substituents selected from halogen, C₁₋₄ alkyl, hydroxyl and -NR'R"; the heteroaryl can be 5-10 membered heteroaryl, such as 5-10 membered heteroaryl containing nitrogen and/or oxygen, such as pyridyl, pyrazolyl and imidazolyl, etc., the heteroaryl can be optionally substituted by 1-4 substituents selected from halogen, C₁₋₄ alkyl, hydroxyl and -NR'R"; the heterocyclyl can be a 4-10 membered heterocyclyl, preferably a 4-10 membered heterocyclyl containing nitrogen and/or oxygen, such as morpholinyl, piperidinyl, piperazinyl, pyrrolidinyl, etc., and the heterocyclyl can be optionally substituted by 1-4 substituents selected from halogen, C₁₋₄ alkyl, hydroxyl and -NR'R".

In a preferred embodiment, in formula I, both A₁ and A₂ are N.

In a preferred embodiment, in formula I, L₁, L₂, L₃ and L₄ are each independently optionally substituted alkylene or optionally substituted -[(CH₂)ₙO]ₘ-(CH₂)ₒ-. Preferably, each of L₁, L₂, L₃ and L₄ is optionally substituted by 1-5 substituents selected from hydroxyl, -NR'R", C₁₋₄ alkoxy and halogen, wherein R' and R" are each independently selected from H and C₁₋₄ alkyl. Preferably, L₁, L₂, L₃ and L₄ are each independently C₁₋₄ alkylene or -[(CH₂)ₙO]ₘ-(CH₂)ₒ-.

In a preferred embodiment, in formula I, n, m and o are each independently an integer of 1-4.

In some preferred embodiments, n is 2, m is 1-4, and o is 2.

In some embodiments, in formula I, L₁, L₂, L₃ and L₄ are all C₁₋₄ alkylene. In some embodiments, L₁, L₂ and L₄ are each independently -[(CH₂)ₙO]ₘ-(CH2)ₒ-, preferably, L₁, L₂ and L₄ are the same group; L₃ is C₁₋₄ alkylene. Preferably, in these embodiments, n, m and o are each independently an integer of 1-4; more preferably, n is 2, m is 1-4, and o is 2.

In a preferred embodiment, in formula I, R₁, R₂, R₃ and R₄ are each independently an alkyl optionally substituted by hydroxyl and/or halogen. Preferably, R₁, R₂, R₃ and R₄ are at least substituted by hydroxyls, preferably substituted by at least one hydroxyl. Preferred numbers of hydroxyl substituents are 1, 2, 3, 4 or 5, more preferably one. Preferably, at least one hydroxyl is located on the second carbon atom of the alkyl from the N atom connecting end. Preferably, the carbon chain length of the alkyl is 1-24 carbon atoms, preferably 8-24 carbon atoms, more preferably 10-20 carbon atoms. In some embodiments, the carbon chain length of the alkyl is 12-18 carbon atoms, preferably, the alkyl has a hydroxyl substitution on the second carbon atom of the alkyl from the N atom connecting end. In some embodiments, R₁, R₂, R₃ and R₄ are each independently -CH₂CH(OH)CH₂R₁₁, wherein the R₁₁ is a C₁₋₂₁ alkyl optionally substituted by halogen, preferably, R₁₁ is C₈₋₁₅ alkyl. In some embodiments, R₁₁ is unsubstituted C₁₋₂₁ alkyl or perfluorinated C₁₋₂₁ alkyl. In a preferred embodiment, R₁, R₂, R₃ and R₄ are the same group.

In a preferred embodiment, in formula I, R₅ₐ, R_{5b}, R₆ₐ, R_{6b}, R₇ₐ, R_{7b}, R₈ₐ and R_{8b} are each independently selected from H and optionally substituted C₁₋₄ alkyl. The C₁₋₄ alkyl is optionally substituted by 1-5 substituents selected from hydroxyl and halogen. More preferably, R₅ₐ, R_{5b}, R₆ₐ, R_{6b}, R₇ₐ, R_{7b}, R₈ₐ and R_{8b} are all H.

In a preferred embodiment, R₉, R₁₀ and R₁₂ are each independently an alkyl optionally substituted by hydroxyl and/or halogen. In some embodiments, R₉, R₁₀ and R₁₂ are each substituted by at least hydroxyl, preferably at least one hydroxyl. Preferred numbers of hydroxyl substituents are 1-5, more preferably one. Preferably, at least one hydroxyl is located on the second carbon atom of the alkyl from the N atom connecting end. Preferably, the carbon chain length of the alkyl is 1-24 carbon atoms, preferably 8-24 carbon atoms, more preferably 10-20 carbon atoms. In some embodiments, the carbon chain length of the alkyl is 12-18 carbon atoms, preferably, the alkyl has a hydroxyl substitution on the second carbon atom of the alkyl from the N atom connecting end. In some embodiments, R₉, R₁₀ and R₁₂ are each independently -CH₂CH(OH)CH₂R₁₁, wherein the R₁₁ is a C₁₋₂₁ alkyl optionally substituted by halogen, preferably, R₁₁ is C₈₋₁₅ alkyl. In some embodiments, R₁₁ is unsubstituted C₁₋₂₁ alkyl or perfluorinated C₁₋₂₁ alkyl. In a preferred embodiment, R₉ and R₁₀ are the same group.

In some preferred embodiments, R₁, R₂, R₃, R₄, R₉ and R₁₀ are all the -CH₂CH(OH)CH₂R₁₁ groups, preferably all are the same group.

In some embodiments, B is R₁₂, and R₁₂ is C₁₋₁₀ alkyl optionally substituted by 1, 2, 3, 4, or 5 hydroxyl groups. Preferably, a hydrogen on the second carbon atom of the alkyl from the N atom connecting end is substituted by one hydroxyl. Preferably, R₁₂ is an unsubstituted C₁₋₄ alkyl or a C₁₋₄ alkyl substituted by 1 or 2 hydroxyl groups.

In some embodiments, B is R₁₂; R₁₂ is a C₁₋₁₀ alkyl optionally substituted by 1-5 hydroxyl groups, preferably, a hydrogen on the second carbon atom of the alkyl from the N atom connecting end is substituted by one hydroxyl; L₁ and L₂ are each independently -[(CH₂)ₙO]ₘ-(CH₂)ₒ- or C₁₋₄ alkylene, preferably, L₁ and L₂ are same group; L₃ is C₁₋₄ alkylene.

In a preferred embodiment, in formula I:
both A₁ and A₂ are N;
R₁, R₂, R₃ and R₄ are each independently C₁₋₂₄ alkyl optionally substituted by hydroxyl; preferably, R₁, R₂, R₃ and R₄ are substituted by at least one hydroxyl; preferably, R₁, R₂, R₃ and R₄ are each independently -CH₂CH(OH)CH₂R₁₁, wherein, the R₁₁ is a C₁₋₂₁ alkyl optionally substituted by halogen, preferably R₁₁ is an unsubstituted C₁₋₂₁ alkyl.
R₅ₐ, R_{5b}, R₆ₐ, R_{6b}, R₇ₐ, R_{7b}, R₈ₐ and R_{8b} are all H;
B is -L₄-N(R₉R₁₀); wherein, L₁, L₂, L₃ and L₄ are all C₁₋₄ alkylene groups, preferably, L₁, L₂, L₃ and L₄ are the same alkylene groups; or L₁, L₂ and L₄ are each independently -[(CH₂)ₙO]ₘ-(CH2)ₒ-, preferably, L₁, L₂ and L₄ are the same group, and L₃ is C₁₋₄ alkylene; R₉ and R₁₀ are each independently C₁₋₂₄ alkyl optionally substituted by hydroxyl and/or halogen; preferably, R₉ and R₁₀ are substituted by at least one hydroxyl; preferably, R₉ and R₁₀ are each independently -CH₂CH(OH)CH₂R₁₁, wherein, the R₁₁ is C₁₋₂₁ alkyl optionally substituted by halogen, preferably, R₁₁ is unsubstituted C₁₋₂₁ alkyl or perfluorinated C₁₋₂₁ alkyl;
or B is R₁₂; wherein, R₁₂ is a C₁₋₁₀ alkyl optionally substituted by 1-5 hydroxyl groups, preferably, a hydrogen on the second carbon atom of the alkyl from the N atom connecting end is substituted by one hydroxyl; L₁ and L₂ are each independently -[(CH₂)ₙO]ₘ-(CH₂)ₒ- or C₁₋₄ alkylene, preferably, L₁ and L₂ are the same group; L₃ is C₁₋₄ alkylene;
n, m, and o are each independently an integer from 1 to 4.

In a preferred embodiment, in formula I: both A₁ and A₂ are N; R₁, R₂ , R₃ and R₄ are each independently -CH₂CH(OH)CH₂R₁₁, wherein, the R₁₁ is an unsubstituted C₅₋₁₈ alkyl; R₅ₐ, R_{5b}, R₆ₐ, R_{6b}, R₇ₐ, R_{7b}, R₈ₐ and R_{8b} are all H; B is R₁₂; and R₁₂ is C₁₋₁₀ alkyl optionally substituted by 1, 2, 3, 4, or 5 hydroxyl groups, preferably is C₁₋₄ alkyl, preferably, a hydrogen on the second carbon atom of the alkyl from the N atom connecting end is substituted by one hydroxyl; L₁ and L₂ are each independently -[(CH₂)ₙO]ₘ-(CH₂)ₒ- or C₁₋₄ alkyl, preferably, L₁ and L₂ are the same group; L₃ is a C₁₋₄ alkylene; n is 2, m is 1-4, and o is 2.

In a preferred embodiment, in formula I: A₁ and A₂ are both N; R₁, R₂, R₃ and R₄ are each independently -CH₂CH(OH)CH₂R₁₁, wherein the R11 is an unsubstituted C₅₋₁₈ alkyl; R₅ₐ, R_{5b}, R₆ₐ, R_{6b}, R₇ₐ, R_{7b}, R₈ₐ and R_{8b} are all H; B is -L₄-N (R₉R₁₀); L₁, L₂ and L₄ are each independently -[(CH₂)ₙO]ₘ-(CH₂)ₒ-; L₃ is a C₁₋₄ alkylene; R₉ and R₁₀ are each independently -CH₂CH(OH)CH₂R₁₁, wherein the R₁₁ is an unsubstituted C₅₋₁₈ alkyl; n is 2, m is 1-4, o is 2.

In a preferred embodiment, the compound I has the following structure:

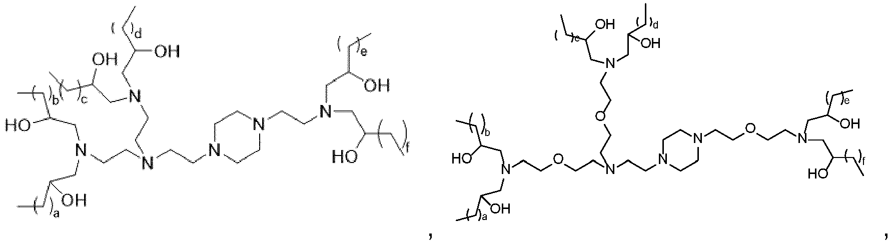

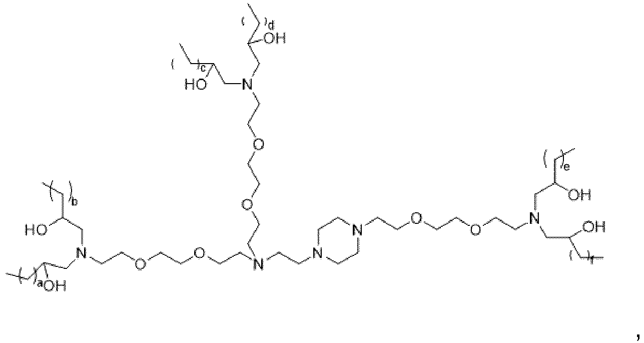

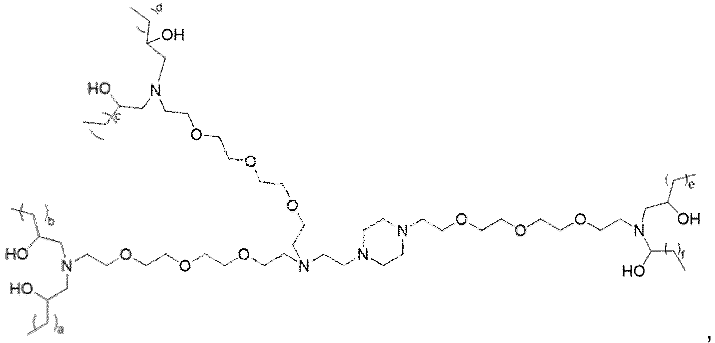

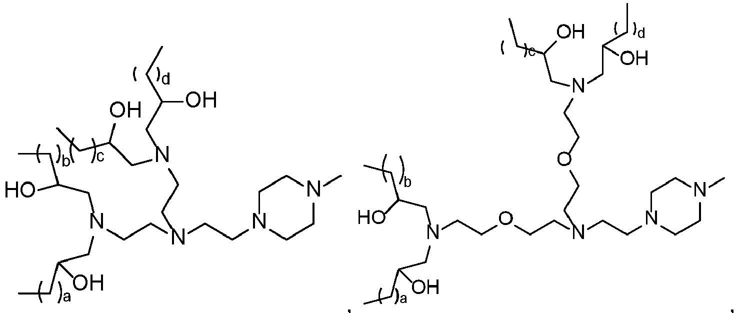

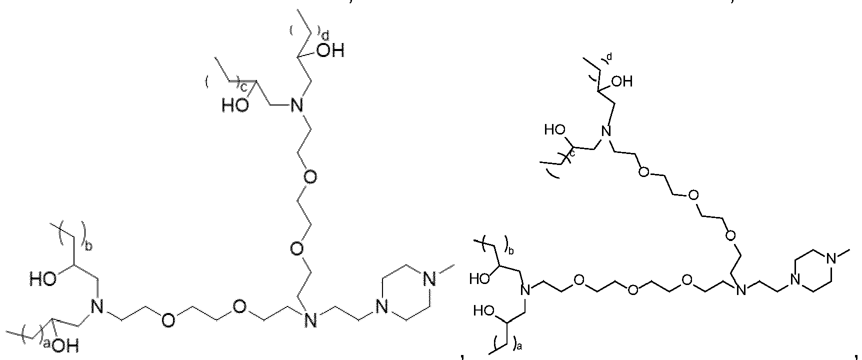

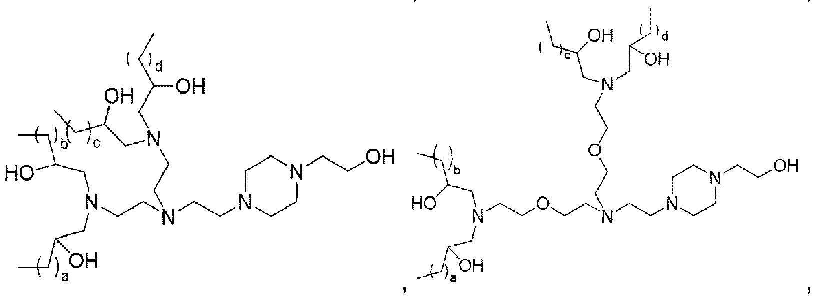

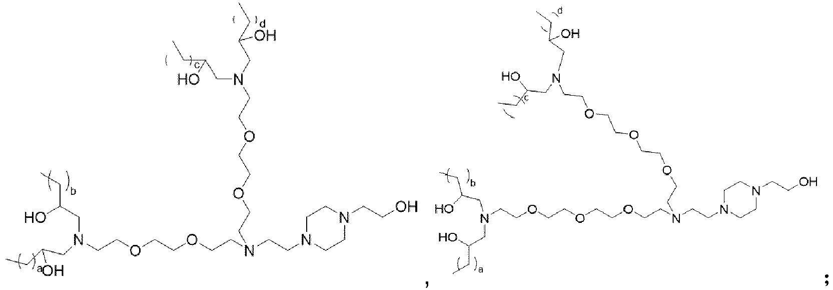

In each structure, a, b, c, d, e and f are each independently an integer from 0 to 20, preferably an integer from 8 to 16, more preferably an integer from 9 to 15. In some embodiments, a, b, c, d, e and f are the same integer selected from 0 to 20, preferably the same integer selected from 8 to 16, more preferably the same integer selected from 9 to 15.

In a preferred embodiment, the compound of formula I is:

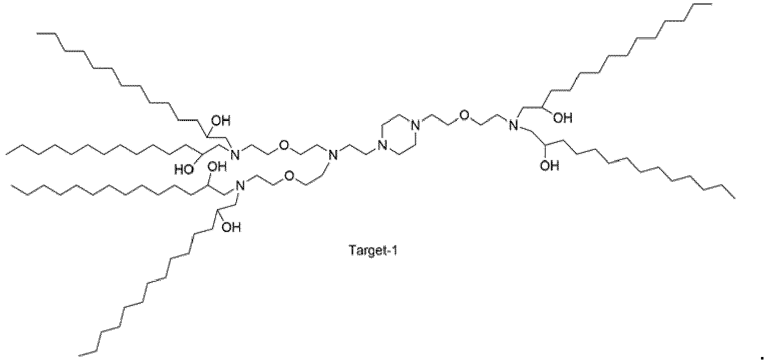

In a preferred embodiment, the compound of formula I is:

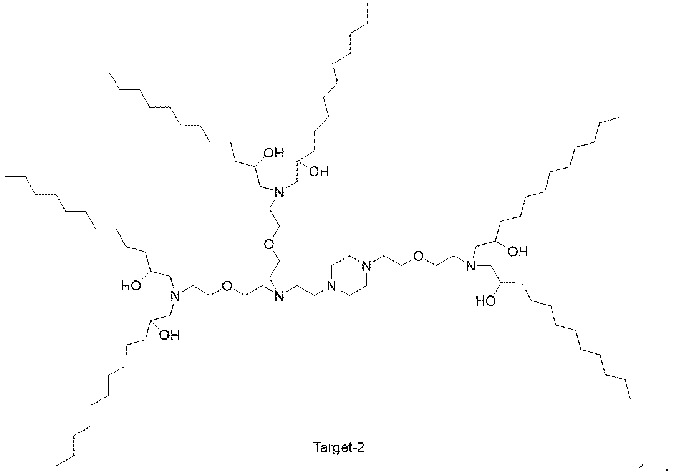

In a preferred embodiment, the compound of formula I is:

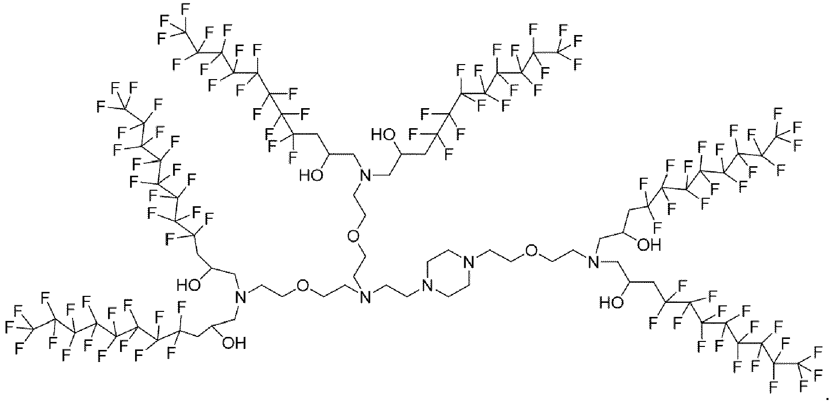

### III. Lipid Nanoparticles

The compound of formula I can be used for preparing the lipid nanoparticles for delivering nucleic acid therapeutic agents or active agents in vivo and in vitro.

In addition to the compounds of formula I of the description, the lipid nanoparticles of the description may also contain one or more auxiliary lipid molecules, one or more cholesterol or cholesterol derivatives and/or one or more polymer conjugated lipid molecules.

Preferably, the auxiliary lipid molecule is a neutral lipid molecule, preferably selected from: DSPC, DPPC, DMPC, DOPC, POPC, DOPE and SM. In a preferred embodiment, the lipid nanoparticles comprise DOPE. In some embodiments, the lipid nanoparticles comprise DSPC.

Preferably, the cholesterol derivative is cholesterol (chol for short, CAS: 57-88-5).

Preferably, in the polymer-conjugated lipid molecule, the polymer is polyethylene glycol. In a preferred embodiment, the polymer-conjugated lipid molecule is selected from PEG-DAG, PEG-PE, PEG-S-DAG, PEG-DSPE, PEG-cer, DMG-PEG and PEG dialkoxypropyl carbamate. Preferably, the lipid nanoparticles contain PEG2000-DSPE. In some embodiments, the lipid nanoparticles comprise DMG-PEG2000.

In a preferred embodiment, in the lipid nanoparticles, when contained, the molar ratio of the compound of formula I, stereoisomer, racemate or pharmaceutically acceptable salt thereof and auxiliary lipid molecule, the cholesterol or cholesterol derivatives and the polymer-conjugated lipid molecules is (60 to 5):(60 to 5):(50 to 5):(10 to 1), preferably (40 to 10):(30 to 10):(50 to 20):(8 to 1). In some embodiments, the molar ratio is (40 to 30):(20 to 10):(50 to 40):(4 to 1).

The average particle size of the lipid nanoparticles of the present description can generally be 30 nm to 150 nm, preferably 40 nm to 150 nm.

In a preferred embodiment, the lipid nanoparticle of the present description contains a therapeutic agent or an active agent, preferably, the therapeutic agent or active agent is a nucleic acid therapeutic agent or active agent. More preferably, the nucleic acid therapeutic agent or active agent is selected from: messenger RNA (mRNA), antisense oligonucleotide (ASO), small interfering RNA (siRNA), microRNA (miRNA), ribozyme and aptamer. In some embodiments, lipid nanoparticles of the description can be used for expressing proteins encoded by mRNA. The protein can be, for example, employed for treatment, prevention or improvement of physiological functions in organisms, including but not limited to antigens, antibodies, and proteins with biological functions known in the art. In other embodiments, the lipid nanoparticles of the description can be used to up-regulate endogenous protein expression by delivering miRNA inhibitors that target a specific miRNA or by delivering a panel of miRNAs that modulate a target mRNA or several mRNAs. In other embodiments, the lipid nanoparticle compositions of the description can be used to downregulate (eg, silence) protein levels and/or mRNA levels of target genes. In some other embodiments, lipid nanoparticles of the description can also be used to deliver mRNA and plasmids to express transgenes. In other embodiments, the lipid nanoparticles of the description can be used to induce pharmacological effects resulting from protein expression, such as increased production of red blood cells by delivery of mRNA of appropriate erythropoietin, or by delivery of mRNA encoding an antigen or antibody of interest to protect against infection. When the lipid nanoparticle of the present description contains a therapeutic agent or an active agent, the mass ratio of the lipid itself to the therapeutic agent or active agent such as mRNA can be in the range of (5 to 20):1.

The lipid nanoparticles of the present description can be prepared by conventional preparation methods in the art. For example, compound I is mixed with auxiliary lipid molecules, the cholesterol and the polymer-conjugated lipid molecules according to a certain molar ratio and dissolved in ethanol to obtain an ethanol lipid solution. The mRNA was dissolved in citrate buffer to obtain an aqueous mRNA solution. The ethanol lipid solution and the mRNA aqueous solution were mixed in a certain volume ratio by using a microfluidic device. The medium was replaced by ultrafiltration, ethanol was removed and volumed with DPBS. Finally, the lipid nanoparticles were filtered through a 0.2 µm sterile filter to obtain LNP.

### IV. Composition

The present description also provides a composition containing the compound of formula I of the present description. In some embodiments, the composition is a pharmaceutical composition, which contains a compound of formula I of the present description, a therapeutic agent or an active agent, and one or more auxiliary lipid molecules, one or more cholesterol or cholesterol derivatives and/or one or more polymer-conjugated lipid molecules.

Preferably, the auxiliary lipid molecule is a neutral lipid molecule, preferably selected from: DSPC, DPPC, DMPC, DOPC, POPC, DOPE and SM. In a preferred embodiment, the lipid nanoparticles comprise DOPE. In some embodiments, the lipid nanoparticles comprise DSPC.

Preferably, the cholesterol derivative is cholesterol.

Preferably, in the polymer-conjugated lipid molecule, the polymer is polyethylene glycol. In a preferred embodiment, the polymer-conjugated lipid molecule is selected from PEG-DAG, PEG-PE, PEG-S-DAG, PEG-DSPE, PEG-cer, DMG-PEG and PEG dialkoxypropyl carbamate. Preferably, the lipid nanoparticles comprise PEG2000-DSPE. In some embodiments, the lipid nanoparticles comprise DMG-PEG2000.

Preferably, the therapeutic or active agent is a nucleic acid therapeutic or active agent. More preferably, the nucleic acid therapeutic agent or active agent is selected from: messenger RNA (mRNA), antisense oligonucleotide (ASO), small interfering RNA (siRNA), microRNA (miRNA), ribozyme and aptamer.

Preferably, in the composition, the lipid nanoparticles according to any embodiment of the present application were formed of the compound of the formula I, one or more auxiliary lipid molecules, one or more cholesterol or cholesterol derivatives and one or more polymer-conjugated lipid molecules, and the lipid nanoparticles encapsulate the therapeutic agent or active agent. Preferably, in the composition, the mass ratio of the lipid nanoparticle itself to the therapeutic agent or active agent such as mRNA may be in the range of (5 to 20): 1.

Preferably, the pharmaceutical composition contains a therapeutically or prophylactically effective amount of the lipid nanoparticles according to any embodiment of the present description and a pharmaceutically acceptable carrier or excipient. In particular, the compounds of formula I of the present description are present in the composition in an amount effective to form lipid nanoparticles and deliver a therapeutically or prophylactically effective amount of a therapeutic or active agent, e.g., for the treatment of a particular target disease or condition. Appropriate concentrations and dosages can be readily determined by those skilled in the art.

In some embodiments, the compositions of the present description further comprise apolipoprotem. As used herein, the term "apolipoprotem" or "lipoprotein" refers to apolipoprotem and its variants and fragments known to those skilled in the art, as well as apolipoprotem agonists or analogs and fragments thereof as described below. Suitable apolipoprotems include, but are not limited to, ApoA-I, ApoA-II, ApoA-IV, ApoA-V and ApoE, and their active polymorphs, isoforms, variants and mutants, and fragments or truncations thereof. In a preferred embodiment, the apolipoprotem is ApoE4.

The pharmaceutical composition of the present description can be formulated into preparations in solid, semi-solid, liquid or gaseous form, such as tablets, capsules, powders, granules, ointments, solutions, suspensions, suppositories, injections, inhalants, gels, microspheres and aerosols. Routes of administration of such pharmaceutical compositions include, but are not limited to, oral, topical, transdermal, inhalation, intraperitoneal, sublingual, buccal, rectal, vaginal and intranasal. The term "intraperitoneal" as used herein includes subcutaneous injection, intravenous, intramuscular, intradermal, intrasternal injection or infusion.

Appropriate pharmaceutically acceptable carriers and excipients can be selected according to specific dosage forms and administration routes. For example, as a solid composition for oral administration, the pharmaceutical composition can be formulated into powders, granules, compressed tablets, pills, capsules, chewing gum, flakes and the like. Such solid compositions generally contain one or more inert diluents or edible carriers. In addition, one or more of the following may be present: binders such as carboxymethylcellulose, ethylcellulose, microcrystalline cellulose, tragacanth or gelatin; excipients such as starch, lactose or dextrin, disintegrants such as alginic acid, sodium alginate, Primogel, corn starch, etc.; lubricants such as magnesium stearate or Sterotex; glidants such as colloidal silicon dioxide; sweeteners such as sucrose or saccharin; flavoring agents such as peppermint , methyl salicylate, or orange flavor; and coloring agent.

When the pharmaceutical composition is in the form of a capsule such as a gelatin capsule, it may contain, in addition to the above materials, a liquid carrier such as polyethylene glycol or oil.

The pharmaceutical compositions of the present description can be prepared by methods well known in the field of pharmacy. For example, pharmaceutical compositions intended to be administered by injection can be prepared by combining lipid nanoparticles of the present description with sterile distilled water or other carriers to form a solution.

The pharmaceutical compositions of this description are administered in a therapeutically effective amount which will vary according to a number of factors including the activity of the particular therapeutic agent employed; the metabolic stability and duration of action of the therapeutic agent; the patient's age, weight, overall health, sex, and diet; route and timing of administration; excretion rates; drug combinations; severity of a particular disorder or condition; and subjects being treated.

### V. Methods of Treatment and Uses

The lipid nanoparticles and pharmaceutical compositions of the description can be used to deliver therapeutic agents or active agents, such as nucleic acids, *in vivo* and *in vitro,* for the treatment or prevention of a disease or condition in a subject.

Accordingly, the present description provides the use of a compound of formula I of the present description in the preparation of a lipid nanoparticle or a pharmaceutical composition for treating or preventing a disease or disorder in a subject, as well as a compound of formula I of the present description, lipid nanoparticle or pharmaceutical composition for treating or preventing a disease or disorder in a subject.

The diseases and disorders described herein may be various diseases and disorders known in the art that are suitable for nucleic acid therapy and prevention, and depend on the specific biological function of the nucleic acid delivered by lipid nanoparticles. In some embodiments, the pharmaceutical composition is a vaccine, and the method includes immunizing an individual so that the individual is immune to a corresponding disease or disorder, including but not limited to infection caused by influenza virus, hepatitis B virus, hepatitis C virus, COVID-19 virus etc. The diseases or conditions also include, for example, tumors, including solid tumors and hematologic tumors, various inflammatory conditions, and the like.

Routes and methods of administration are well known in the art and described above, such as including but not limited to oral, topical, transdermal, inhalation, intraperitoneal, sublingual, buccal, rectal, vaginal and intranasal. In some embodiments, the intraperitoneal route of administration includes: subcutaneous injection, intravenous, intramuscular, intradermal, intrasternal injection or infusion.

The description also includes the use of a compound of formula I of the description for the delivery of a therapeutic agent or active agent, such as a nucleic acid, or for the preparation of a reagent for the delivery of a therapeutic agent or active agent, such as a nucleic acid. The present description also provides a method of delivering a therapeutic agent or an active agent such as a nucleic acid *in vivo* or *in vitro*, the method comprising encapsulating the therapeutic agent or an active agent in a lipid nanoparticle made of a compound of formula I, and delivering the therapeutic agent or active agent via the lipid nanoparticles or compositions containing the lipid nanoparticles. The nucleic acid may be as described in any of the embodiments herein. In some embodiments, the therapeutic agent or active agent (such as a nucleic acid) is delivered to a target site of interest (such as a cell, a tissue (diseased tissues such as tumor tissue, etc.), organs) via the lipid nanoparticle or a composition containing the lipid nanoparticle. In some embodiments, the cells are immune cells, including but not limited to T cells, dendritic cells (DC cells) or tumor infiltrating lymphocytes (TIL) and the like.

### VI. The preparation method of formula I compound

The compound of formula I of the present description can be prepared by following general synthetic schemes I and II:

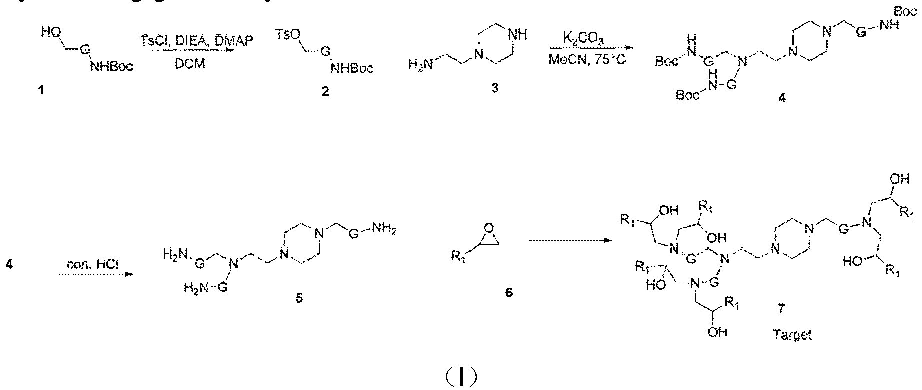

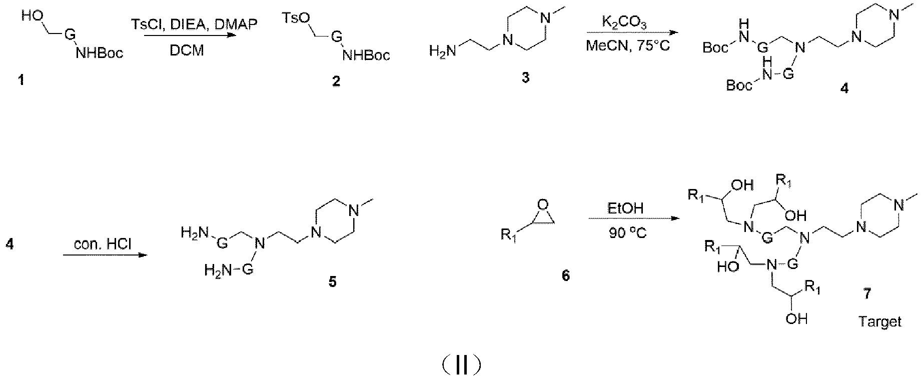

Each R₁ in the above general synthetic schemes I and II can be independently selected from H, halogen, optionally substituted alkyl, optionally substituted alkenyl, optionally substituted cycloalkyl, optionally substituted heterocyclyl , optionally substituted cycloalkenyl, optionally substituted cycloalkynyl, optionally substituted aryl, optionally substituted heteroaryl, optionally substituted alkoxy, optionally substituted alkoxycarbonyl, optionally substituted acyl, -NR'R", carboxy, nitro, cyano, or alkylsulfoxide; wherein R' and R" are each independently selected from H or optionally substituted alkyl. Each G is independently selected from optionally substituted alkylene, optionally substituted -[(CH₂)ₙO]ₘ-(CH2)ₒ-, optionally substituted alkenylene and optionally substituted alkynylene; When synthesizing other compounds of formula I, compounds having corresponding groups can be used as reactants to replace the corresponding reactants in the above-mentioned synthetic schemes I and II.

The present description will be further described below in the form of specific examples. It should be understood that these examples are merely illustrative and are not intended to limit the scope of the present description. Unless otherwise specified, the methods and materials used in the examples are conventional materials and methods in the art.

### Example

### Example 1: the synthesis of target compound

### 1. Synthesis of A-C12, A-C14, A-C16, A-C18

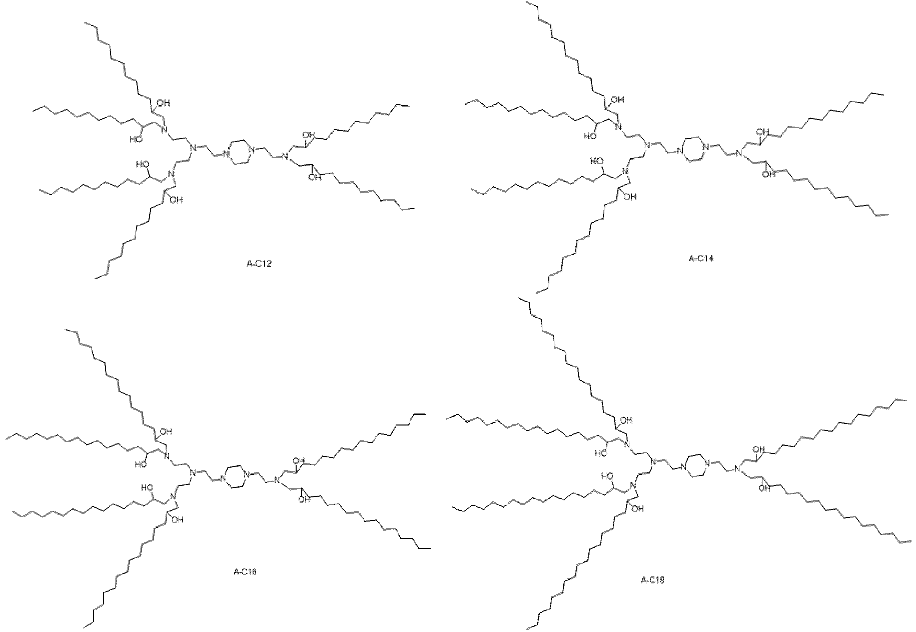

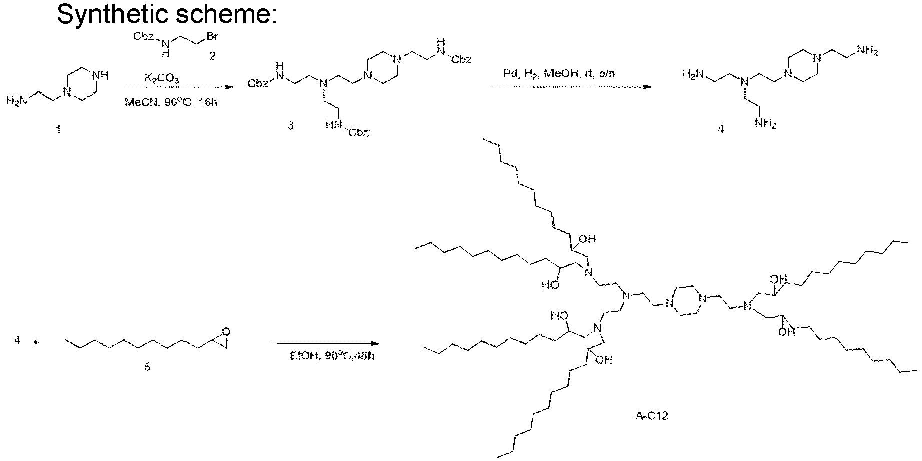

### Step 1: Synthesis of Compound 3 ((2-(4-(2-(2-(((Benzyloxycarbonyl)amino)ethyl)aminoethyl)piperazinylethyl)c arbamate)

The mixture of 2-(piperazin-1-yl)ethan-1-amine(1 g, 7.75 mmol, 1.0 eq), benzyl (2-bromoethyl) carbamate(7.9 g, 31.0 mmol, 4.0 eq), K₂CO₃ (6.4 g, 46.5 mmol, 6.0 eq) was dissolved in anhydrous acetonitrile (50 ml), heated and stirred at 80 °C overnight. TLC (MeOH/DCM=1/10) showed complete disappearance of the starting amine. Insoluble salts or other impurities were removed by suction filtration under reduced pressure, and the filter cake was washed with DCM. The organic phases were combined and concentrated by rotary evaporation to obtain the crude product. The crude product was purified by combiFlash (silica gel column 40 g, DCM/MeOH (containing 10% ammonia)=100/1-100/7, volume ratio). The pure product fractions were evaporated to obtain compound 3 (4 g, 78.2% yield) as a colorless oil. ESI: [M+1]: 661.1.

### Step 2: Synthesis of Compound 4 (N 1-(2-aminoethyl)-n 1-(2-(4-(2-aminoethyl)piperazin-1-yl)ethyl)ethylenediam ine)

(2-(4-(2-(2-(((Benzyloxycarbonyl)amino)ethyl)aminoethyl)piperazinyleth yl)carbamate (4.0 g, 6.0 mmol, 1.0 eq) was dissolved in 40 ml of anhydrous methanol, Pd/C (10%, 400 mg) was added, and reacted overnight under hydrogen atmosphere. After the reaction was completed, vacuum filtration, filter cake was washed with a small amount of methanol, and the filtrate was concentrated under reduced pressure to obtain compound 4 (1.2 g, 76.9% yield).ESI: [M+1]: 259.1.

### Step 3: Synthesis of 1, 1', 1", 1"-((2-(4-(2-(2-(2-(2-(2-(2-hydroxydodecyl)aminoethyl)piperazin-1-yl)ethyl)az odimethyl)bis(2,1-diyl)bis(azotriyl)tetra(dodecan-2-ol)

Compound 4 (100 mg, 0.387 mmol, 1.0 eq), 1,2-epoxydodecane (712 mg, 3.87 mmol, 10.0 eq), EtOH (1 ml) were mixed well. Heated to reflux and stirred for 48 h. The reaction solution was concentrated by rotary evaporation to obtain the crude product. The crude product was purified by combiFlash (silica gel column 20 g, DCM/MeOH (containing 10% ammonia)=100/1-100/7, volume ratio). The pure product fractions were evaporated to obtain the target product A-C12 (200 mg, 37.8% yield) as a colorless oil.

The 1,2-epoxydodecane in the above step 3 was replaced by 1,2-epoxytetradecane, 1,2-epoxyhexadecane and 1,2-epoxyoctadecane respectively to synthesize the target product A-C14, A-C16, A-C18.

### 2. Synthesis of B-C12, B-C14, B-C16, B-C18

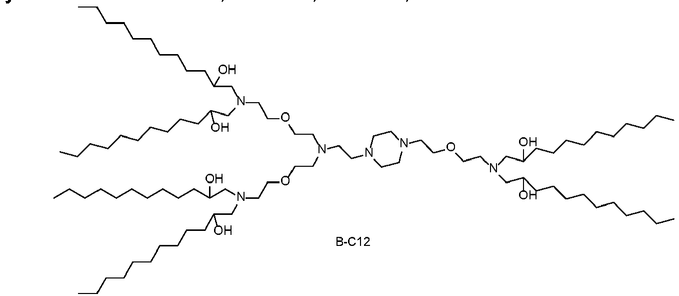

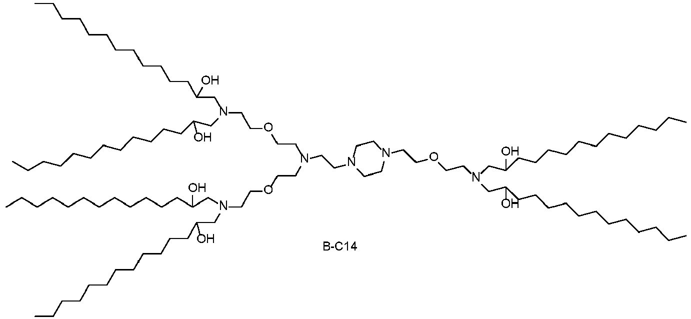

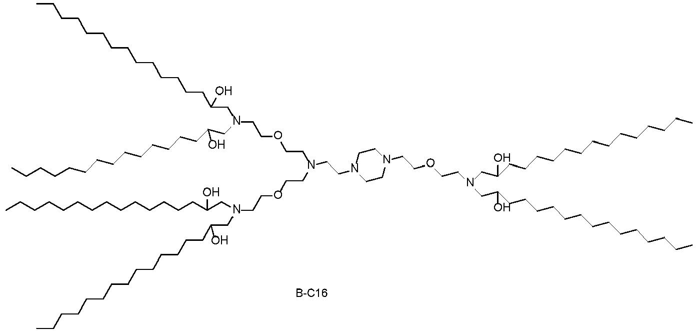

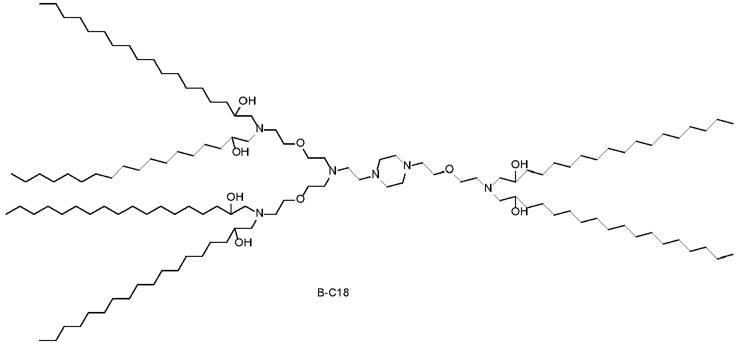

### Synthetic steps:

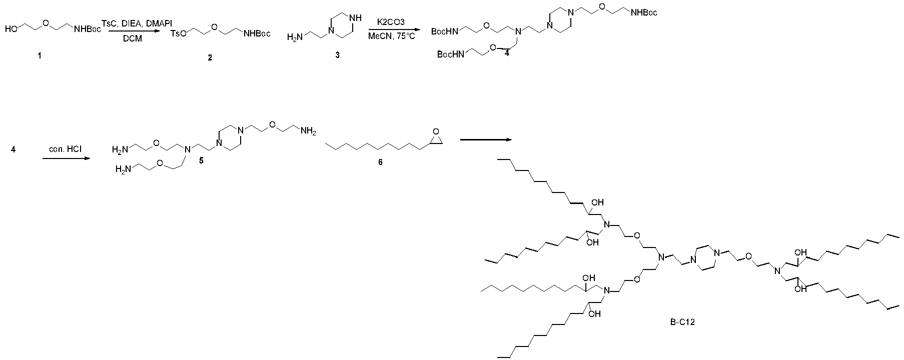

### Step 1: Synthesis of Compound 2 ((2-(2-((tert-butoxycarbonyl)amino)ethoxy)ethyl-4-methylbenzenesulfonate)

Tert-butyl (2-(2-hydroxyethoxy) ethyl) carbamate (20 g, 97.56 mmol, 1 eq), DMAP (12 g, 97.50 mmol, 1 eq), DIEA (25 g, 193 mmol, 2 eq) were mixed in DCM (300 ml) and stirred at room temperature until all material was dissolved. At room temperature, TsCI (22 g, 115.79, 1.2 eq) was slowly added in batches to the DCM solution to obtain a light yellow solution. After stirring overnight at room temperature, TLC showed complete disappearance of the starting alcohol. The reaction solution was diluted with DCM (200 ml), and washed with water (100 ml*3), diluted hydrochloric acid 1N (100 ml*3), saturated NaHCOs (100 ml*3) and brine (40 ml*2), respectively. The organic layers were combined and dried over anhydrous sodium sulfate. The organic phase was concentrated by rotary evaporation to obtain 33 g of light yellow oily liquid. It was directly used in the next reaction without further purification. ESI: [M+1]: 360.1.

### Step 2: Synthesis of Compound 4 (tert-butyl(2-(2-(4-(11-(2-(2-((tert-butoxycarbonyl)amino)ethoxy)ethyl)-2,2-di methyl-4-oxo-3,8-dioxa-5,11-diazatridecane-13-yl)piperazin-1-yl)ethoxy)ethy l)carbamate)

The mixture of 2-(piperazin-1-yl)ethan-1-amine(1 g, 7.75 mmol, 1.0 eq), 2-(2-((tert-butoxycarbonyl)amino)ethoxy)ethyl 4-methyl phenylsulfonate (11.1 g, 31.0 mmol, 4.0 eq), K₂CO₃ (6.4 g, 46.5 mmol, 6.0 eq) was dissolved in anhydrous acetonitrile (50 ml), heated and stirred at 80°C overnight. TLC (MeOH/DCM=1/10) showed complete disappearance of the starting amine. Insoluble salts or other impurities were removed by suction filtration under reduced pressure, and the filter cake was washed with DCM. The organic phases were combined and concentrated by rotary evaporation to obtain the crude product. The crude product was purified by combiFlash (silica gel column 40 g, DCM/MeOH (containing 10% ammonia)=100/1-100/7, volume ratio). The pure product fractions were evaporated to obtain compound 4 (2.6 g, 49% yield) as a colorless oil. ESI: [M+1]: 692.0.

### Step 3: Synthesis of Compound 5 (2-(2-aminoethoxy)-N-(2-(2-aminoethoxy)ethyl)-N-(2-(4-(2-(2-aminoethyl oxy)ethyl)piperazin-1-yl)ethyl)ethan-1-amine)

Compound 4 (tert-butyl(2-(2-(4-(11-(2-(2-((tert-butoxycarbonyl)amino)ethoxy)ethyl)-2,2-di methyl-4-oxo-3,8-dioxa-5,11-diazatridecane-13-yl)piperazin-1-yl)ethoxy)ethy l)carbamate) (2.6 g, 3.77 mmol, 1.0 eq) was dissolved in 10 ml of 1,4-dioxane, 6 ml of concentrated hydrochloric acid was added, and the mixture was stirred overnight at room temperature. The reaction solution was concentrated by rotary evaporation to dryness, dissolved in 10 ml of water, Na₂CO₃ was added to adjust the pH to alkaline, and stirred for 2 h. The reaction solution was concentrated until completely dry, 100 ml THF and 10 g anhydrous sodium sulfate were added and stirred for 3 h. Suction filtration under reduced pressure to remove insoluble salts, and the filter cake was washed with THF (50 ml*3). The organic phases were combined and concentrated by rotary evaporation to obtain compound 5 as light yellow oil (1.2 g, 82% yield). ESI: [M+1]: 391.1.

### Step 4: Synthesis of B-C12(19-(2-(4-(2-(2-(di(2-hydroxydodecyl)amino)ethoxy)ethyl)piperazin-1-y l)ethyl)-13,15-bis(2-hydroxydodecyl)-16,22-dioxa-13,19,25-triazaheptakadec ane-11,27-diol)

Compound 5 (100 mg, 0.256 mmol, 1.0 eq), 1,2-epoxydodecane (470 mg, 2.56 mmol, 10.0 eq), EtOH (1 ml) were mixed well, and stirred at reflux for 48 h. TLC showed compound 5 was disappeared. The reaction solution was concentrated by rotary evaporation to obtain the crude product. The crude product was purified by combiFlash (silica gel column 20 g, DCM/MeOH (containing 10% ammonia)=100/1-100/7, volume ratio). The pure product fractions were evaporated to obtain the product B-C12 (Target-2, 200 mg, 52.2% yield) as a colorless oil.

The 1,2-epoxydodecane in step 3 was replaced by 1,2-epoxytetradecane, 1,2-epoxyhexadecane, and 1,2-epoxyoctadecane respectively to synthesize the target product B-C14 (Target-1), B-C16 and B-C18.

### 3. Synthesis of C-C12, C-C14, C-C16, C-C18

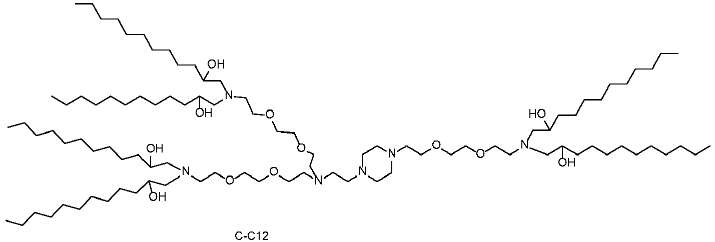

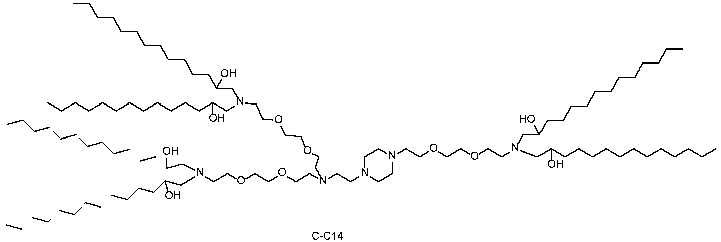

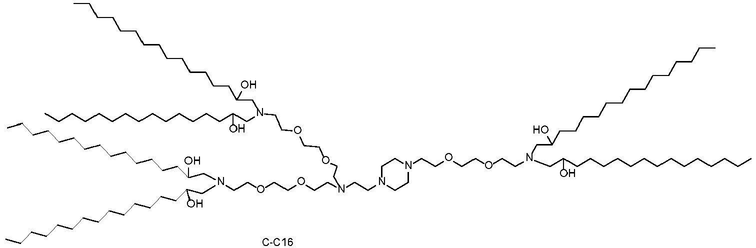

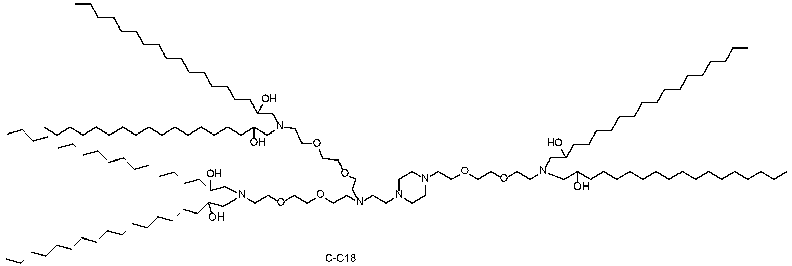

The synthesis steps are as follows:

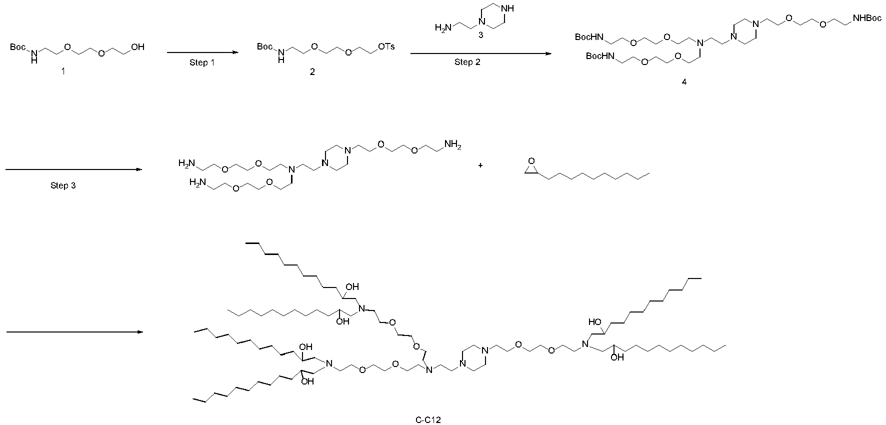

C-C12 was synthesized by the synthetic method of compound B-C12, except that tert-butyl (2-(2-hydroxyethoxy)ethyl)carbamate was replaced by (2-(2-(2-hydroxyethoxy)ethoxy)ethyl) tert-butyl carbamate. The 1,2-epoxydodecane was replaced by 1,2-epoxytetradecane, 1,2-epoxyhexadecane and 1,2-epoxyoctadecane respectively to prepare C-C14 and C-C16 and C-C18.

### 4. Synthesis of D-C12, D-C14, D-C16, D-C18

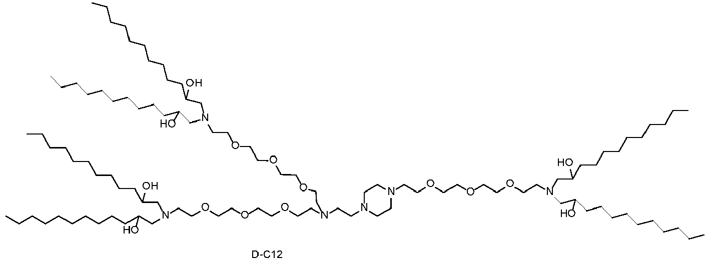

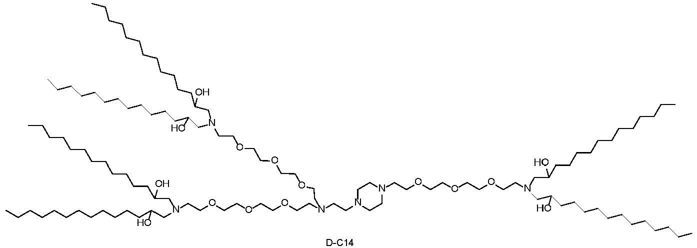

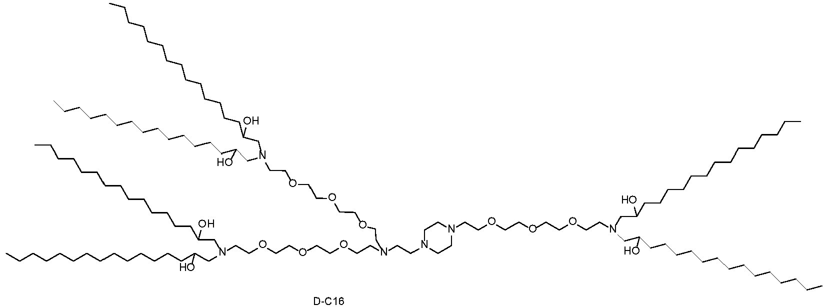

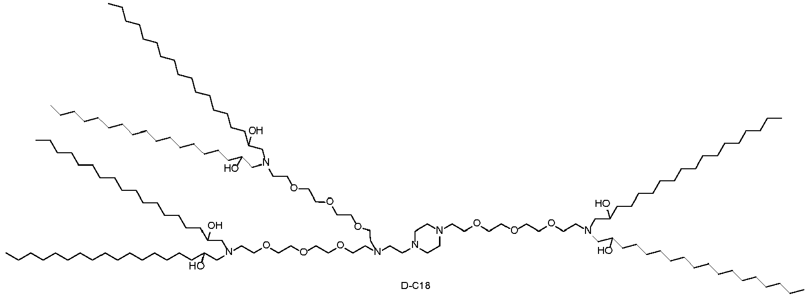

The synthesis steps are as follows:

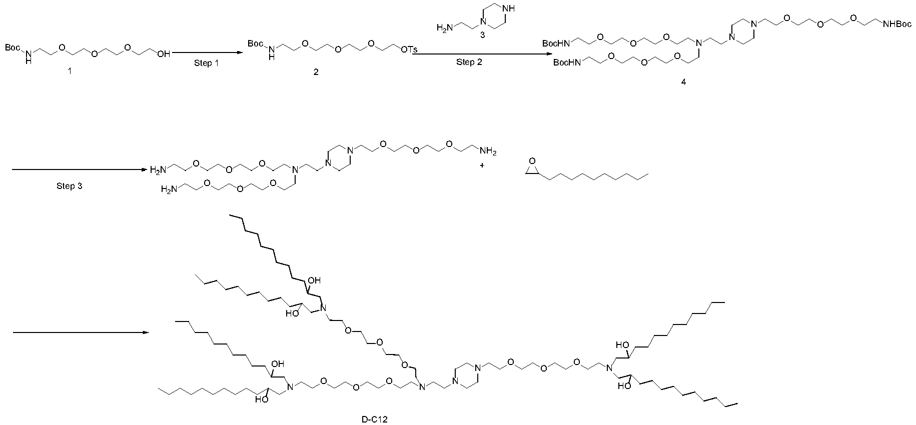

D-C12 was synthesized using the method of compound B-C12 except that tert-butyl(2-(2-hydroxyethoxy)ethyl)carbamate was replaced by tert-butyl (2-(2-(2-(2-hydroxyethoxy) ethoxy) ethoxy) ethyl) carbamate. The 1,2-epoxydodecane was replaced by 1,2-epoxytetradecane, 1,2-epoxyhexadecane and 1,2-epoxyoctadecane respectively to prepare D-C14 and D-C16 and D-C18.

### 5. Synthesis of E-C12, E-C14, E-C16, E-C18

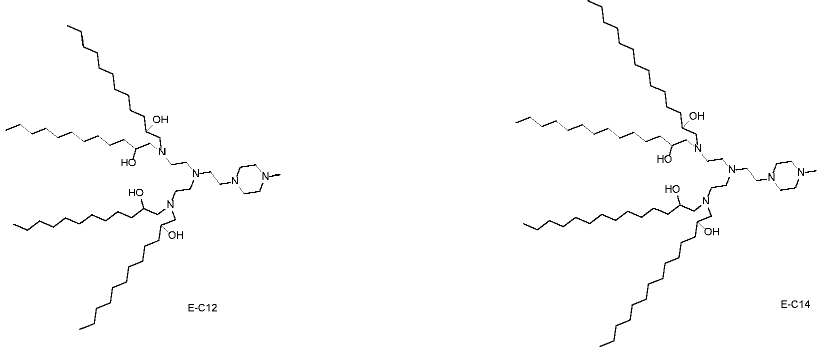

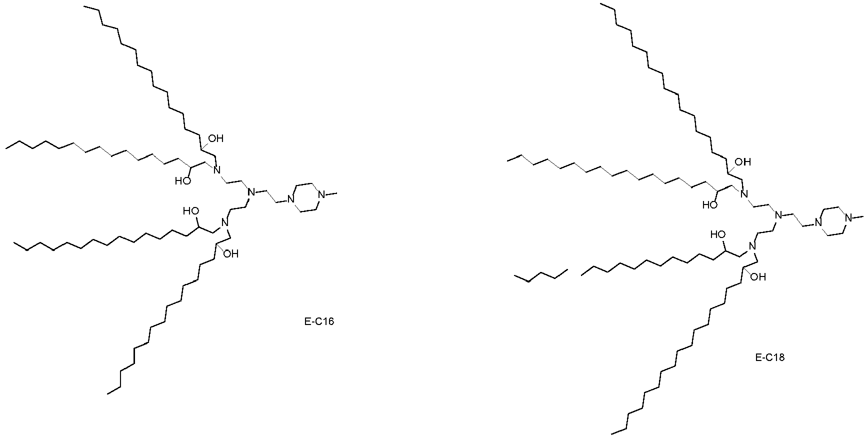

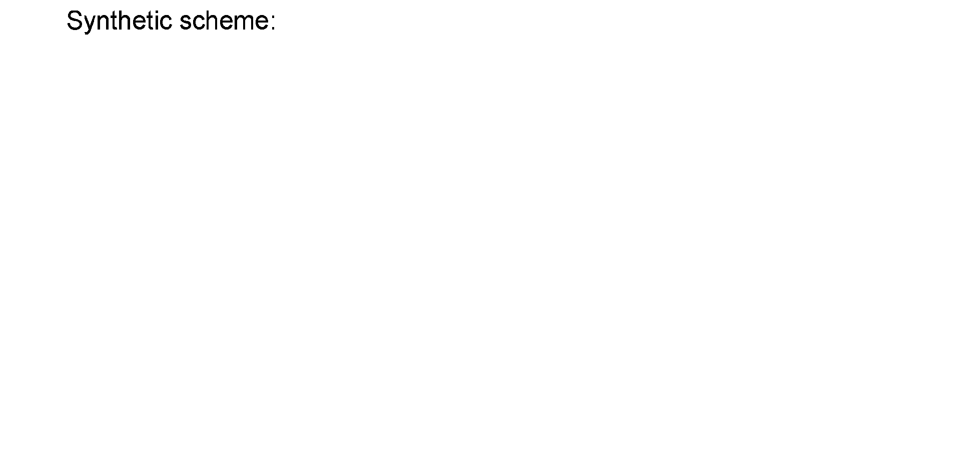

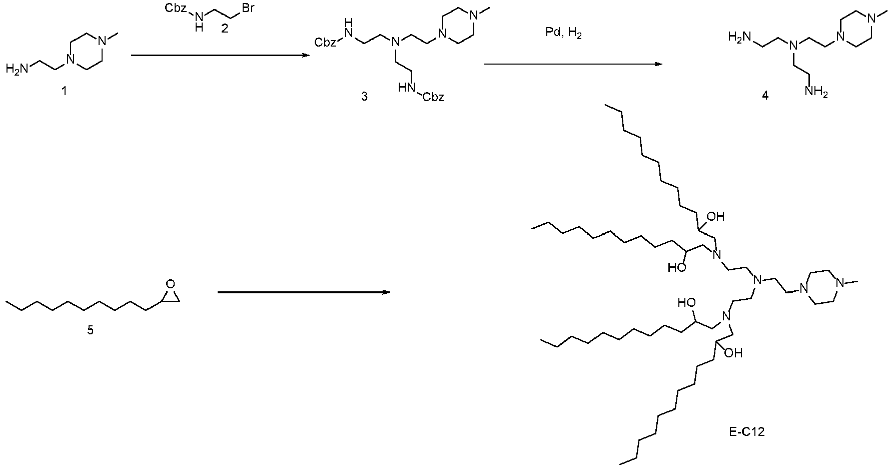

E-C12 was synthesized by the synthetic method of A-C12, except that 2-(piperazin-1-yl)ethan-1-amine was replaced by 4-methyl-1-piperazineethylamine. The 1,2-epoxydodecane was replaced by 1,2-epoxytetradecane, 1,2-epoxyhexadecane and 1,2-epoxyoctadecane respectively to prepare E-C14, E-C16 and E-C18.

### 6. Synthesis of F-C12, F-C14, F-C16, F-C18

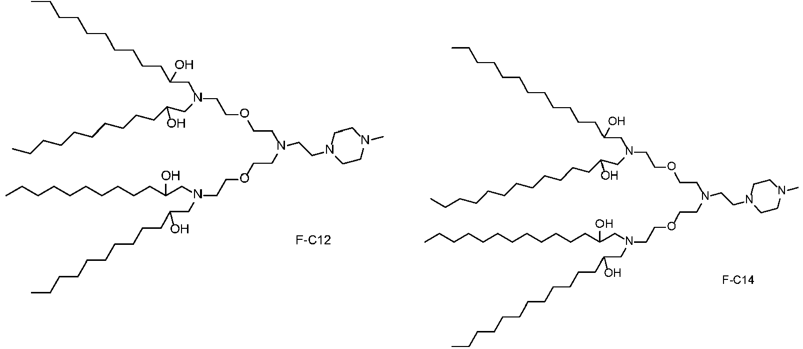

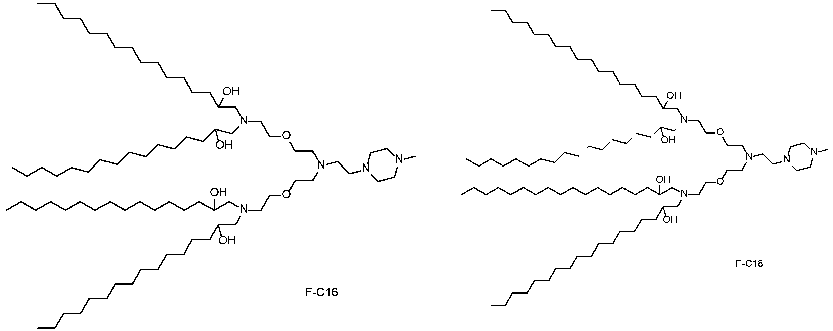

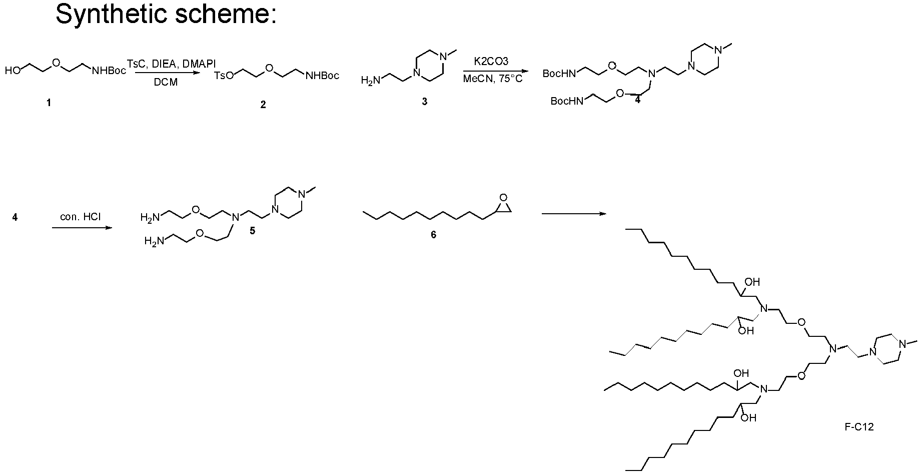

F-C12 was synthesized by the synthetic method of B-C12, except that 2-(piperazin-1-yl)ethan-1-amine was replaced by 4-methyl-1-piperazineethylamine. The 1,2-epoxydodecane was replaced by 1,2-epoxytetradecane, 1,2-epoxyhexadecane and 1,2-epoxyoctadecane respectively to prepare F-C14, F-C16 and F-C18.

### 7. Synthesis of G-C12, G-C14, G-C16, G-C18

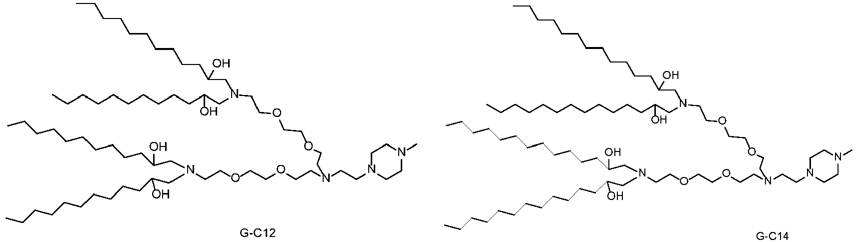

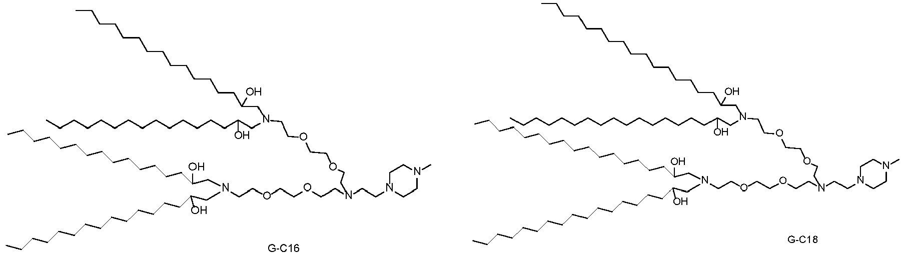

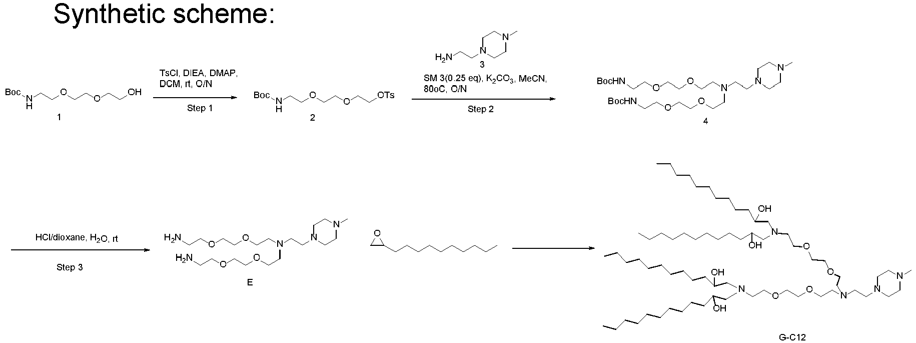

G-C12 was synthesized by the synthetic method of C-C12, except that 2-(piperazin-1-yl)ethan-1-amine was replaced by 4-methyl-1-piperazineethylamine. The 1,2-epoxydodecane was replaced by 1,2-epoxytetradecane, 1,2-epoxyhexadecane and 1,2-epoxyoctadecane respectively to prepare G-C14 and G-C16 and G-C18.

### 8. Synthesis of H-C12, H-C14, H-C16, H-C18

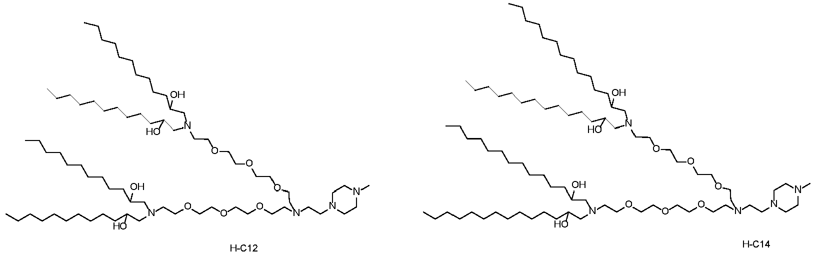

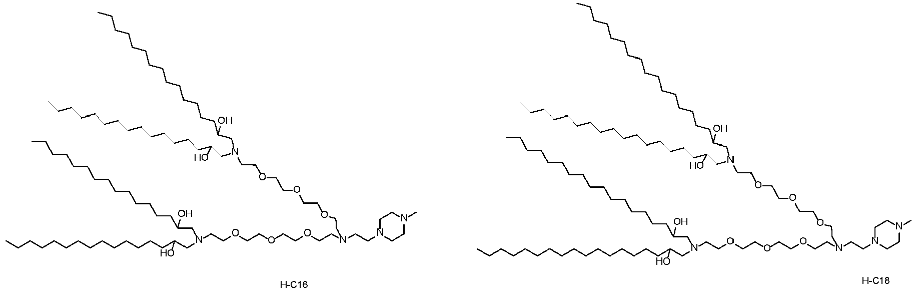

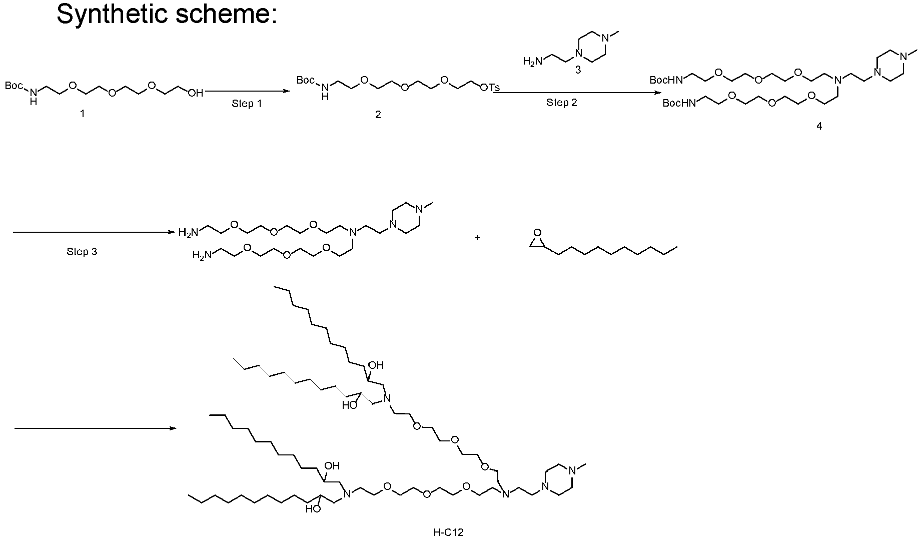

H-C12 was synthesized by the synthetic method of D-C12, except that 2-(piperazin-1-yl)ethan-1-amine was replaced by 4-methyl-1-piperazineethylamine. The 1,2-epoxydodecane was replaced by 1,2-epoxytetradecane, 1,2-epoxyhexadecane and 1,2-epoxyoctadecane respectively to prepare H-C14, H-C16 and H-C18.

### 9. Synthesis of I-C12, I-C14, I-C16, I-C18, J-C12, J-C14, J-C16, J-C18, K-C12, K-C14, K-C16, K-C18, L-C12, L-C14, L-C16, L-C18

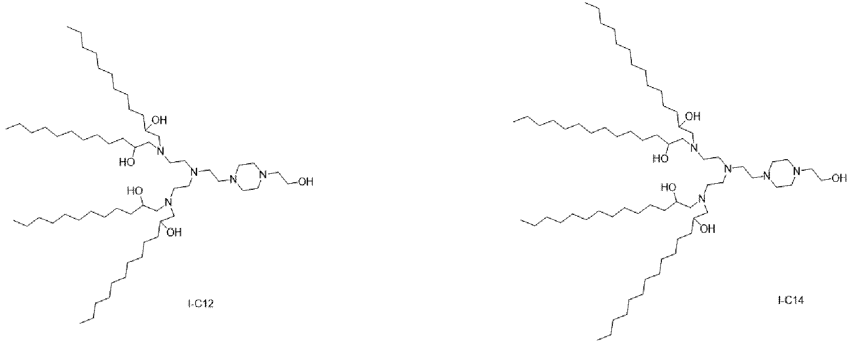

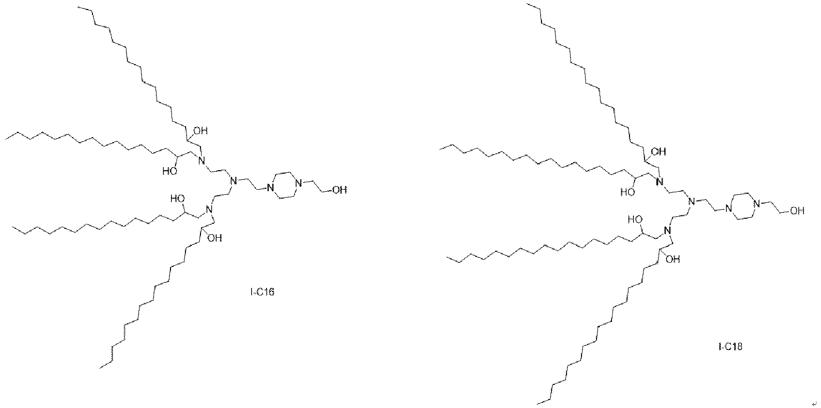

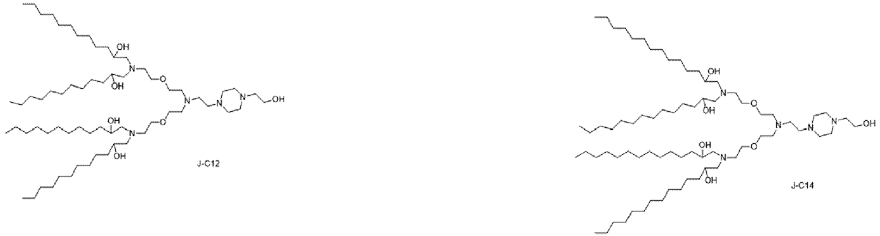

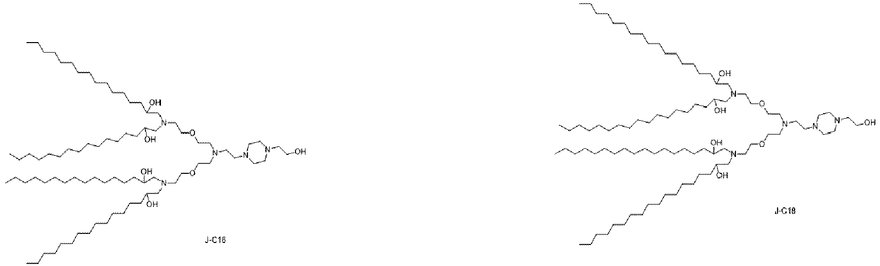

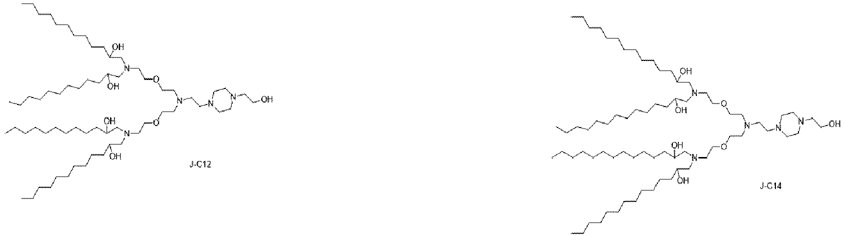

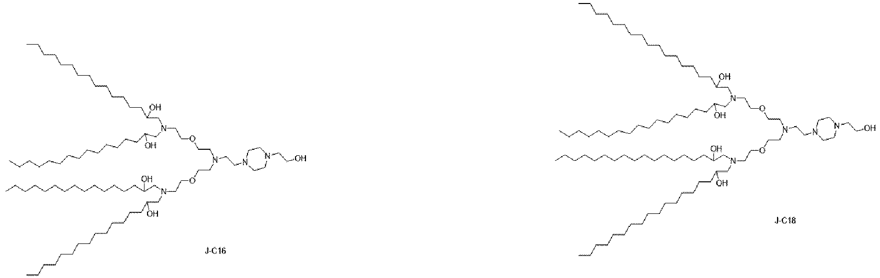

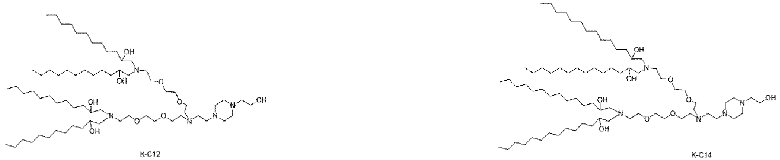

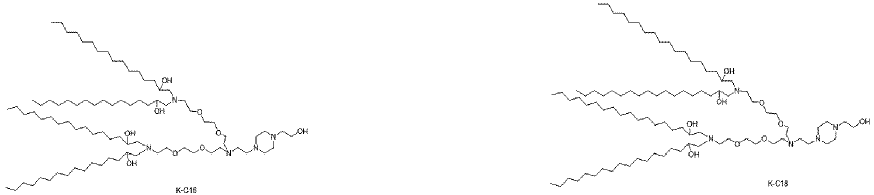

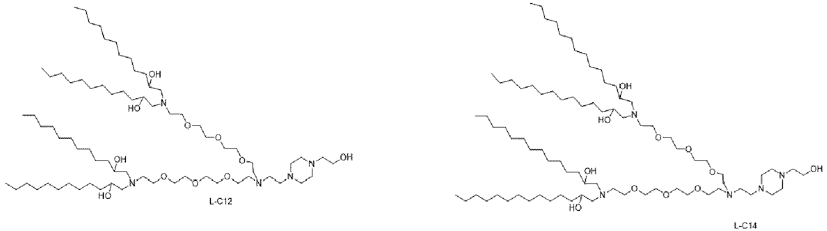

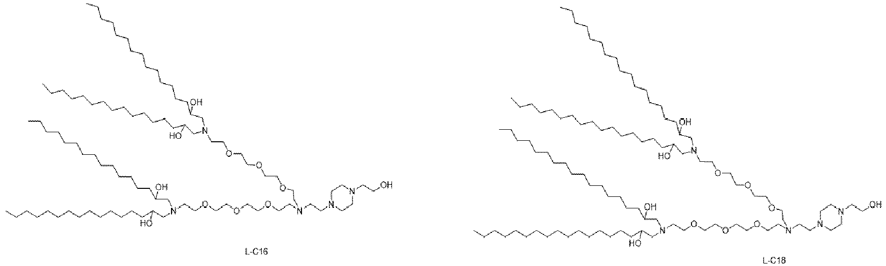

Intermediate compound 4 is a shared raw material for the synthesis of I, J, K, and L, and its synthetic route is as follows:

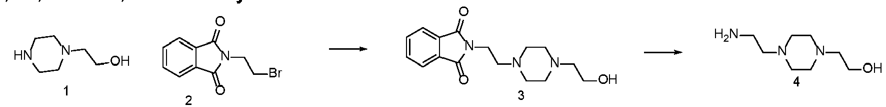

### Synthesis of compound 3

N-(2-bromoethyl)phthalimide (8.78 g, 34.56 mmol, 0.9 eq), anhydrous potassium carbonate (12.53 g) were added into a 500 ml eggplant-shaped flask, and protected with nitrogen. In addition, compound 1 (5 g, 38.41 mmol, 1 eq) was weighed and dissolved in 50 mL of anhydrous acetonitrile (10 times m/v) under nitrogen protection, and added into an eggplant-shaped bottle with a syringe, and stirred. Heated and stirred overnight at 80°C. The reaction was monitored by TLC (TLC condition: DCM: MeOH=10:1), and after uv showed that the reaction was completed, the reaction solution was filtered with a sand core funnel. The filter cake was washed three times with 50 mL of acetonitrile, and all filtrates were collected. 10 g of silica gel was added to the filtrate, and rotary evaporation was conducted until the silica gel is dry. The crude product was purified by combiFlash (silica gel column 40 g, DCM/MeOH (containing 10% ammonia)=100/1-100/7, volume ratio). The target compound 3 (8.22 g, yield 70.56%) was obtained. ESI: [M+1]: 304.2.

### Synthesis of compound 4

Compound 3 (8.2 g, 27.03 mmol, 1 eq) was added into an eggplant-shaped flask containing 160 mL of ethanol and stirred, then 26.05 mL of 35% concentrated ammonia water was added, and refluxed at 90°C overnight. A white suspension was obtained the next day. TLC monitoring (TLC conditions: DCM: MeOH (NH₃) = 10: 1, uv and ninhydrin color comparison) confirmed the exhaustion of the raw materials, and the reaction solution was cooled to room temperature. 10 g of diatomaceous earth was charged into a sand core funnel, and the reaction solution was filtered to collect the filtrate. 200 mL of acetonitrile was added to the filtrate, and a large amount of white precipitate was obtained. The white precipitate was filtered through celite, and the filtrate was collected. The filtrate was concentrated and dried to obtain the target compound 4 (4.3 g, 92% yield) as a colorless oil, ESI: [M+1]: 174.1.

The compounds of series I, J, K and L were synthesized according to the compounds of series A, B, C and D respectively, and the specific synthesis method will not be described again.

MS and HNMR of the compound synthesized above are shown in the following table:

| Sample number | MS:[M+1] | HNMR |
|---|---|---|
| A-C12 | 1364.5 | 1 HNMR (400 M, CDCl3) δ ppm: 3.76-3.45 (m, 8 H), 2.88-2.27 (m, 36 H), 1.53-1.10 (m, 108 H) , 0.94-0.90 (m, 18 H) . |
| A-C14 | 1532.5 | 1 HNMR (400 M, CDCl3) δ ppm: 3.77-3.46 (m, 8 H), 2.89-2.25 (m, 36 H), 1.55-1.11 (m, 132 H) , 0.95-0.91 (m, 18 H) . |
| B-C12 | 1496.4 | 1 HNMR (400 M, CDCl3) δ ppm: 3.68-3.45 (m, 21 H), 2.91-2.38 (m, 36H), 1.53-1.10 (m, 108 H) , 0.95-0.92 (m, 18 H) |
| B-C14 | 1664.5 | 1 HNMR (400 M, CDCl3) δ ppm: 3.69-3.47 (m, 21 H), 2.93-2.39 (m, 36 H), 1.53-1.10 (m, 133 H), 0.96-0.93 (m, 18 H) . |
| C-C12 | 1628.5 | 1 HNMR (400 M, CDCl3) δ ppm: 3.84-3.51 (m, 32 H), 2.85-2.42 (m, 34 H), 1.52-1.11 (m, 110 H), 0.97-0.94 (m, 18 H) . |
| C-C14 | 1796.8 | 1 HNMR (400 M, CDCl3) δ ppm: 3.86-3.52 (m, 32 H), 2.86-2.43 (m, 34 H), 1.53-1.13 (m, 133 H), 0.96-0.91 (m, 18 H). |
| D-C12 | 1761.0 | 1 HNMR (400 M, CDCl3) δ ppm: 3.80-3.53 (m, 44 H), 2.87-2.40 (m, 36 H), 1.51-1.10 (m, 108 H), 0.95-0.92 (m, 18 H). |
| D-C14 | 1929.0 | 1 HNMR (400 M, CDCl3) δ ppm: 3.81-3.52 (m, 44 H), 2.88-2.41 (m, 36 H), 1.52-1.12 (m, 132 H), 0.96-0.92 (m, 18 H). |
| E-C12 | 967.0 | 1 HNMR (400 M, CDCl3) δ ppm: 3.79-3.47 (m, 10 H), 2.87-2.25 (m, 24 H), 1.47-1.10 (m, 73 H), 0.94-0.91 (m, 12 H) . |
| E-C14 | 1079.2 | 1 HNMR (400 M, CDCl3) δ ppm: 3.81-3.46 (m, 10 H), 2.86-2.24 (m, 24 H), 1.48-1.09 (m, 88 H), 0.95-0.90 (m, 12 H). |
| F-C12 | 1055.0 | 1 HNMR (400 M, CDCl3) δ ppm: 3.79-3.47 (m, 16 H), 2.87-2.25 (m, 28 H), 1.48-1.11 (m, 72 H), 0.95-0.91 (m, 12 H). |
| F-C14 | 1167.0 | 1 HNMR (400 M, CDCl3) δ ppm: 3.81-3.46 (m, 16 H), 2.88-2.24 (m, 28 H), 1.49-1.10 (m, 88 H), 0.96-0.92 (m, 12 H). |
| G-C12 | 1143.1 | 1 HNMR (400 M, CDCl3) δ ppm: 3.79-3.47 (m, 20 H), 2.87-2.25 (m, 31 H), 1.48-1.11 (m, 73 H), 0.95-0.91 (m, 12 H). |
| G-C14 | 1255.2 | 1 HNMR (400 M, CDCl3) δ ppm: 3.81-3.49 (m, 20 H), 2.85-2.27 (m, 31 H), 1.50-1.12 (m, 80 H) , 0.95-0.90 (m, 12 H) . |
| H-C12 | 1231.0 | 1 HNMR (400 M, CDCl3) δ ppm: 3.80-3.48 (m, 30 H), 2.87-2.26 (m, 30 H), 1.49-1.10 (m, 72 H) , 0.96-0.92 (m, 12 H). |
| H-C14 | 1343.0 | 1 HNMR (400 M, CDCl3) δ ppm: 3.81-3.49 (m, 30 H), 2.88-2.27 (m, 30 H), 1.47-1.08 (m, 88 H), 0.97-0.92 (m, 12 H) . |
| I-C14 | 1109.0 | 1 HNMR (400 M, CDCl3) δ ppm: 3.70-3.40 (m, 10 H), 2.96-2.34 (m, 36 H), 1.55-1.20 (m, 88 H), 0.95-0.90 (m, 12 H) . |

### Example 2: Preparation and detection of lipid nanoparticles (LNP preparation)

The ionizable lipid compound of Example 1 was dissolved in ethanol with DOPE, cholesterol and DSPE-PEG2000 (all purchased from AVT (Shanghai) Pharmaceutical Technology Co., Ltd.) at a molar ratio of 35:16:46.5:2.5 to prepare ethanol lipid solution, and enhanced green fluorescent protein (EGFP) mRNAs (Shanghai Maxima Pharmaceutical Engineering Co., Ltd., JN-4201) were diluted in 20 mM citrate buffer (pH=6.1) to obtain mRNA aqueous solution. Ethanol lipid solution and mRNA aqueous solution were mixed at a volume ratio of 1:3 by using a microfluidic device (Micro&Nano (Shanghai) Instrument Technology Co., Ltd., model: INano^{™}L), LNPs were prepared with a weight ratio of approximately 10:1 between total lipids and mRNAs. The medium was replaced by ultrafiltration, ethanol was removed and volumed with DPBS. Finally, the lipid nanoparticles were filtered through a 0.2 µm sterile filter to obtain a LNP formulation encapsulating eGFP mRNA using ionizable lipid/DOPE/cholesterol/DSPE-PEG2000 (35/16/46.5/2.5 mol%), the concentration of mRNAs being 0.002 mg/mL-0.5 mg/mL.

Using Malvern Zetasizer Nano ZS (Malvern UK), the size and polydispersity index PDI of lipid nanoparticles were measured at 173° backscatter angle. The test results were shown in Table 1.

### Example 3: Transfection of Lipid nanoparticle (LNP preparation) into cell in vitro

The description is based on peripheral blood mononuclear cells PBMC (Shanghai Oribiotech Co., Ltd.), human peripheral blood leukemia T cell Jurkat (ATCC), tumor infiltrating lymphocyte TIL (isolated from resected tumor tissue of a liver cancer patient, the preparation method refers to CN114763530A) , mouse bone marrow-derived dendritic cells DC2.4 (Cell Bank of the Chinese Academy of Sciences), and Chinese hamster ovary cells CHO (ATCC) were used as verified cell lines for liposome transfection. PBMC, Jurkat, and TIL cells grew in suspension, while DC2.4 and CHO cells grew by adherent culture. All the operation steps of cell culture must strictly abide by the prescribed procedures, and sterile reagents is used to perform aseptic operations under a sterile workbench. The main steps are as follows:
1) Cell recovery: the preserved cell cryo preservation solution was taken out of the -80°C refrigerator, and was immediately put in a 37°C water bath for rapid and continuous shaking, and was completely melted within one minute. Fresh medium was added to the 15 mL centrifuge tube in advance, and the cell solution was added to the centrifuge tube in a biological safety cabinet. A centrifuge tube of the same quality was taken and placed symmetrically in a centrifuge, centrifuged at a low speed at 1200 rpm for 5 min. After centrifugation, the supernatant was removed, and an appropriate amount of medium was slowly added along the tube wall. After the cells were evenly dispersed by blowing gently, the cell suspension was pipette and added to the culture bottle prepared in advance. The state and distribution of the cells in the early stage of recovery were observed under a microscope. The culture bottle was placed in a 5% CO₂ incubator and incubated at 37°C, and the recovery time was recorded.
2) Cell subculture: Under the microscope, if the cell density in the field of view reaches 80%-90%, the cells can be subcultured. Adherent cells need to be digested with trypsin. In order to avoid the residual serum in the culture medium to reduce the activity of trypsin, DPBS solution was added to wash once after removing the original culture medium. After remove the DPBS solution, trypsin was added to degrade the protein at the junction between cells. This separates the cells for further culture. The cells were gently shaked evenly and put into the incubator for digestion for 2 min. Immediately after the digestion was completed, an appropriate amount of medium was added to the original petri dish to terminate the digestion. Finally, the cells were carefully blowed off using a pipette and blowed until they are evenly dispersed, and then inoculated and subcultured at a certain ratio according to the experimental requirements. Suspension cells do not require trypsinization.

The medium used for each cell line and the specific experimental steps are as follows:
Transfection of PBMC cells
1) Coating antibodies: 6-well plates were pre-coated, CD3 and CD28 antibodies were taken out from the -20°C refrigerator, and melted for later use. The above two antibodies were added into the DPBS solution at a concentration of 5 µg/mL, mixed well, added into a six-well plate at 1 mL/well, put in a 37°C incubator, and waited 4 hours for later use.
2) PBMC recovery: the frozen PBMC cells were taken out from the -80°C refrigerator, put in a 37°C water bath to quickly thaw the cells, added to the preheated medium after thawing, centrifuged at 1200 rpm for 5 min, the supernatant was discarded, and an appropriate amount of AIM-V medium with 500U/ml IL-2 was added to resuspend PBMC.
3) the DPBS coated with the antibody was removed with a pipette, the cell suspension was transferred to a six-well plate pre-coated with the antibody at 4 mL per well, placed in a 5% CO₂ incubator at 37°C for 3-5 days for activation and culture.
4) According to the experimental arrangement, PBMC cells were plated on a 24-well plate 30 minutes in advance on the day of cell transfection. After counting the cells, the cell number and cell viability were recorded, and the cells suspension were taken at 5×10⁵ cells per well, centrifuged at 1200 rpm for 5 min, and the supernatant was discarded. The volume of serum-free Opti-MEM medium that needs to be added was calculated according to 300 µL per well, gently pipette evenly, and plated in 24-well plates for subsequent transfection experiments. ApoE4 can be added according to the experimental arrangement, with 0.5 ug/well.
5) The LNP preparation prepared in example 2 was diluted with Opti-MEM medium until the mRNA concentration is 1 ug/ml.
6) 200 ul of the diluted mixture was evenly added along the top of each well, and each well contained 200 ng of mRNA. The plate was placed in a 37°C, 5% CO₂ incubator for activation and culture.
7) After 24 h and 48 h, cell viability, EGFP positive rate, and average fluorescence intensity data were obtained by cell fluorescence imaging and flow cytometry.

### Transfection of Jurkat cells

1) Jurkat recovery: the frozen Jurkat cells were taken out from the -80°C refrigerator, put in a 37°C water bath to quickly thaw the cells, added to the preheated medium after thawing, centrifuged at 1200 rpm for 5 min, the supernatant was discarded, and an appropriate amount of RPMI-1640 medium with 10% FBS was added to resuspend recoveried Jurkat, and placed in a 5% CO₂ incubator at 37°C for activation and culture.
2) According to the experimental arrangement, Jurkat cells were plated on a 24-well plate 30 minutes in advance on the day of cell transfection. After counting the cells, the cell number and cell viability were recorded, and the cell suspension were taken at 5×10⁵ cells per well, centrifuged at 1200 rpm for 5 min, and the supernatant was discarded. The volume of serum-free Opti-MEM medium that needs to be added was calculated according to 300 µL per well, gently pipette evenly, and plated in 24-well plates for subsequent transfection experiments. ApoE4 can be added according to the experimental arrangement, 0.5 ug/well.

Subsequent steps are the same as the transfection protocol for PBMC cells.

### Transfection of TIL cells

1) TIL recovery: the frozen TIL cells were taken out from the -80°C refrigerator, put in a 37°C water bath to quickly thaw the cells, added to the preheated medium after thawing, centrifuged at 1200 rpm for 5 min, the supernatant was discarded, and an appropriate amount of medium was added to resuspend the recovered TIL, and placed in a 5% CO₂ incubator at 37°C for activation and culture.
2) According to the experimental arrangement, TIL cells were plated on a 24-well plate 30 minutes in advance on the day of cell transfection. After counting the cells, the cell number and cell viability were recorded, the cell suspension were taken at 5×10⁵ cells per well, centrifuged at 1200 rpm for 5 min, and the supernatant was discarded. The volume of serum-free Opti-MEM medium that needs to be added was calculated according to 300 µL per well, gently pipette evenly, and plated in 24-well plates for subsequent transfection experiments. ApoE4 can be added according to the experimental arrangement, 0.5 ug/well.

Subsequent steps are the same as the transfection protocol for PBMC cells.

### Transfection of DC2.4 cells

1) DC2.4 cells recovery: the frozen DC2.4 cells were taken out from the -80°C refrigerator, put in a 37°C water bath to quickly thaw the cells, added to the preheated medium after thawing, centrifuged at 1200 rpm for 5 min, the supernatant was discarded, and an appropriate amount of DMEM medium with 10% FBS was added to resuspend the recovered DC2.4, and placed in a 5% CO₂ incubator at 37°C for activation and culture.
2) According to the experimental arrangement, DC2.4 cells were plated on a 24-well plate one day in advance for cell transfection. Through cell counting, the number of cells and cell viability were recorded, the cell suspension were taken at 2×10⁵ cells per well, then a certain volume of DMEM medium containing 10% FBS was added and mixed evenly, 1 mL of mixture was added to each well for subsequent transfection experiment.
3) After 24 hours, the medium was gently removed 30 minutes in advance by a pipette, 1 mL DPBS was added to each well for washing and then removed, then 300 µL serum-free Opti-MEM medium was added to each well for subsequent transfection experiments.

Subsequent steps are the same as the transfection protocol for PBMC cells.

### Transfection of CHO cells

1) CHO cell recovery: the frozen CHO cells were taken out from the -80°C refrigerator, put in a 37°C water bath to quickly thaw the cells, added to the preheated medium after thawing, centrifuged at 1200 rpm for 5 min, the supernatant was discarded, and an appropriate amount of medium was added to resuspend the recovered CHO, and placed in a 5% CO₂ incubator at 37°C for activation and culture.
2) According to the experimental arrangement, CHO cells were plated on a 24-well plate one day in advance for cell transfection. Through cell counting, the number of cells and cell viability were recorded, the cell suspension were taken at 2×10⁵ cells per well, then a certain volume of medium was added and mixed evenly, 1 mL of mixture was added to each well for subsequent transfection experiment.
3) After 24 hours, the medium was gently removed 30 minutes in advance by a pipette, 1 mL DPBS was added to each well for washing and then removed, then 300 µL serum-free Opti-MEM medium was added to each well for subsequent transfection experiments.

Subsequent steps are the same as the transfection protocol for PBMC cells.

### Experimental results

Through flow cytometry analysis, the cell viability and the positive rate of EGFP were obtained. Table 1 shows the characteristics of the lipid nanoparticles prepared in Example 2, as well as the expression level of EGFP mRNA on DC2.4, which was normalized based on the sample with the highest fluorescence intensity.

**Table 1**

| Sample Number | Particle Size | PDI | Entrapmen t Efficiency | Cell Viability | Positive Rate | Fluorescenc e Intensity |
|---|---|---|---|---|---|---|
| A-C12 | 91.6 | 0.173 | 89.80% | 87.1% | 73.3% | 0.84 |
| A-C14 | 97.1 | 0.107 | 80.31% | 95.4 | 92.3% | 0.30 |
| A-C16 | 90.8 | 0.183 | 90.30% | 90.0% | 66.3% | 0.80 |
| A-C18 | 96.9 | 0.144 | 90.56% | 93.0% | 61.9% | 0.87 |
| B-C12 | 102.9 | 0.147 | 89.93% | 85.0% | 83.0% | 0.20 |
| B-C14 | 90.5 | 0.133 | 95.21% | 95.0% | 90.2% | 1.00 |
| B-C16 | 101.7 | 0.071 | 85.25% | 90.2% | 65.7% | 0.49 |
| B-C18 | 108.3 | 0.118 | 94.79% | 89.6% | 68.6% | 0.27 |
| C-C12 | 92.5 | 0.070 | 95.24% | 94.0% | 64.4% | 0.71 |
| C-C14 | 92.2 | 0.171 | 92.82% | 94.6 | 93.6% | 0.75 |
| C-C16 | 94.1 | 0.108 | 90.26% | 97.6% | 85.2% | 0.87 |
| C-C18 | 107.6 | 0.134 | 81.92% | 89.6% | 86.1% | 0.74 |
| D-C12 | 90.5 | 0.073 | 91.80% | 90.8% | 65.6% | 0.07 |
| D-C14 | 97.2 | 0.168 | 92.36% | 91.3 | 96.7% | 0.95 |
| D-C16 | 91.6 | 0.117 | 86.56% | 91.1% | 71.7% | 0.82 |
| D-C18 | 103.0 | 0.105 | 88.54% | 81.5% | 64.4% | 0.45 |
| E-C12 | 96.9 | 0.133 | 95.02% | 84.8% | 56.4% | 0.69 |
| E-C14 | 85.2 | 0.119 | 85.62% | >99.0 | 84.3% | 0.45 |
| E-C16 | 76.9 | 0.107 | 91.51% | 94.4% | 59.9% | 0.71 |
| E-C18 | 84.5 | 0.136 | 92.86% | 98.5% | 65.2% | 0.14 |
| F-C12 | 77.7 | 0.133 | 92.31% | 87.1% | 63.9% | 0.28 |
| F-C14 | 82.4 | 0.131 | 95.21% | 93.2 | 73.5% | 0.33 |
| F-C16 | 91.9 | 0.099 | 92.39% | 96.8% | 60.3% | 0.17 |
| F-C18 | 77.2 | 0.081 | 80.61% | 93.5% | 61.5% | 0.67 |
| G-C12 | 89.3 | 0.172 | 89.95% | 92.9% | 83.1% | 0.29 |
| G-C14 | 86.4 | 0.180 | 84.02% | >99.0% | 67.5 | 0.30 |
| G-C16 | 78.2 | 0.102 | 85.40% | 94.5% | 79.6% | 0.54 |
| G-C18 | 99.8 | 0.195 | 80.79% | 95.2% | 63.9% | 0.63 |
| H-C12 | 95.9 | 0.136 | 94.13% | 86.0% | 74.3% | 0.78 |
| H-C14 | 94.8 | 0.099 | 91.67% | 91.7% | 66.8% | 0.52 |
| H-C16 | 95.5 | 0.162 | 86.56% | >99.0% | 59.0% | 0.77 |
| H-C18 | 73.8 | 0.143 | 80.61% | 97.4% | 81.9% | 0.38 |
| I-C12 | 83.8 | 0.114 | 88.38% | 98.5% | 63.9% | 0.05 |
| I-C14 | 120.3 | 0.180 | 87.59% | 97.0% | 62.7% | 0.05 |
| I-C16 | 96.3 | 0.147 | 91.60% | 81.3% | 68.7% | 0.06 |
| I-C18 | 72.3 | 0.109 | 83.06% | 86.0% | 66.4% | 0.04 |
| J-C12 | 99.7 | 0.105 | 92.88% | 81.8% | 62.6% | 0.04 |
| J-C14 | 126.1 | 0.121 | 83.58% | >99.0% | 59.1% | 0.06 |
| J-C16 | 104.1 | 0.077 | 81.08% | 85.2% | 62.0% | 0.04 |
| J-C18 | 93.7 | 0.163 | 88.56% | 83.6% | 60.9% | 0.04 |
| K-C12 | 75.9 | 0.075 | 92.83% | >99.0% | 62.8% | 0.07 |
| K-C14 | 122.7 | 0.155 | 87.00% | 82.3% | 59.8% | 0.03 |
| K-C16 | 76.0 | 0.185 | 95.74% | 93.4% | 60.2% | 0.04 |
| K-C18 | 124.2 | 0.134 | 92.77% | >99.0% | 74.7% | 0.05 |
| L-C12 | 88.4 | 0.181 | 93.38% | 84.9% | 58.1% | 0.07 |
| L-C14 | 101.5 | 0.126 | 91.54% | 99.0% | 60.0% | 0.04 |
| L-C16 | 111.5 | 0.085 | 92.34% | 97.2% | 64.8% | 0.06 |
| L-C18 | 124.0 | 0.182 | 91.70% | 98.6% | 59.7% | 0.02 |

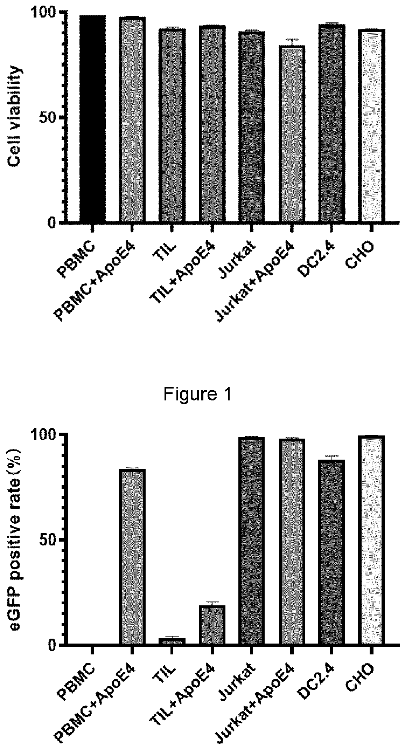

Figure 1 shows the cell viability obtained by flow cytometry analysis after the LNP preparation prepared with B-C14 was transfected in vitro. It can be seen from Figure 1. Cells such as PBMCs, TIL, Jurkat, DC2.4 and CHO have high viability, indicating that the preparation has no cytotoxicity. ApoE4 added during transfection did not affect cell viability.

Table 2 shows the expression level of EGFP mRNA on PBMCs after the LNP preparation prepared in Example 2 was added with ApoE4 to transfect PBMCs, and the normalization process was performed based on the sample with the highest fluorescence intensity.

**Table 2**

| Sample Number | Cell Viability | Positive Rate | Fluorescence Intensity |
|---|---|---|---|
| A-C12 | 89.70% | 73.30% | 0.72 |
| A-C14 | 91.20% | 72.30% | 0.76 |
| A-C16 | 90.00% | 76.30% | 0.81 |
| A-C18 | 85.00% | 81.90% | 0.87 |
| B-C12 | 85.00% | 83.00% | 0.81 |
| B-C14 | 90.00% | 85.50% | 1 |
| B-C16 | 91.20% | 86.70% | 0.89 |
| B-C18 | 87.50% | 78.60% | 0.65 |
| C-C12 | 89.30% | 56.50% | 0.41 |
| C-C14 | 83.40% | 93.60% | 0.31 |
| C-C16 | 87.60% | 85.20% | 0.27 |
| C-C18 | 89.30% | 86.10% | 0.43 |
| D-C12 | 85.60% | 65.60% | 0.27 |
| D-C14 | 88.70% | 96.70% | 0.25 |
| D-C16 | 82.20% | 71.70% | 0.32 |
| D-C18 | 83.30% | 64.40% | 0.41 |
| E-C14 | 86.50% | 64.60% | 0.12 |
| F-C14 | 83.20% | 63.70% | 0.16 |
| G-C14 | 79.40% | 63.40% | 0.1 |
| H-C14 | 81.70% | 62.10% | 0.07 |

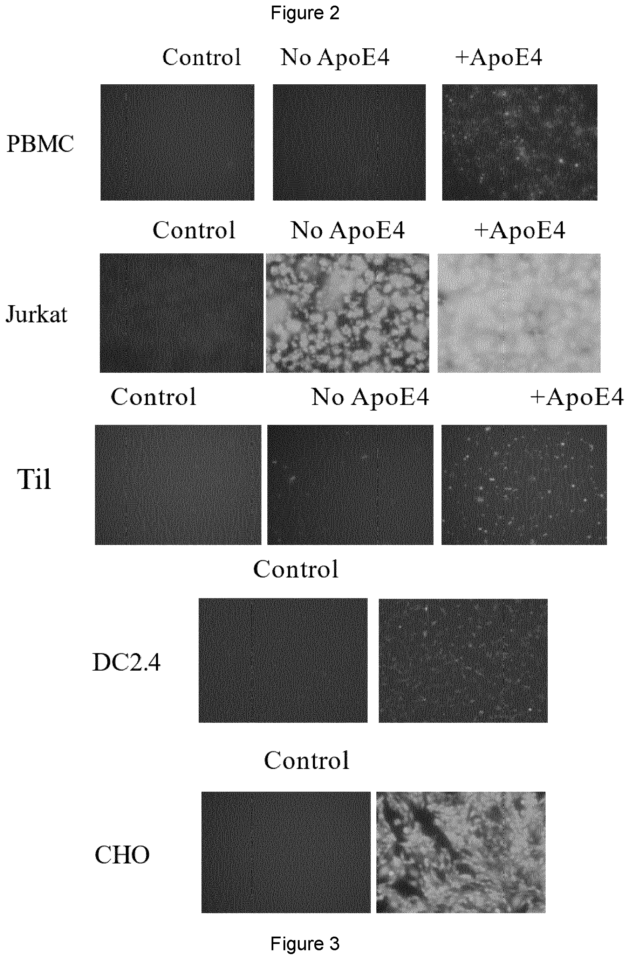

Figures 2 and 3 show the transfection efficiency into cells of LNP preparations prepared with B-C14 without or with the addition of AopE4. In the absence of AopE4, the LNP preparation had high transfection efficiency on Jurkat, DC2.4 and CHO cells, but low transfection efficiency on two T cells, PBMC and TIL (Figure 2). With ApoE4, the transfection efficiency of both PBMC and TIL cells were increased, and the transfection efficiency of PBMC reached over 75% (Figure 3).

### Example 4: In vitro stability test of lipid nanoparticle (LNP preparation)

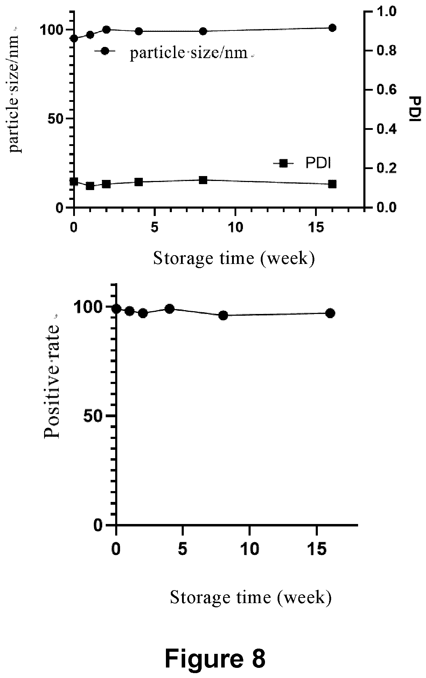

The preparation from Example 2 was stored at 4°C-8°C. Samples were taken every other week to test the transfection performance of the samples on DC2.4, the size of the nanoparticles and the polydispersity index PDI. The whole process lasted 16 weeks. The results of LNP prepared with B-C14 were shown in Figure 8. It can be seen that the particle size, PDI and cell transfection efficiency remained in the initial state when stored at 4-8 °C for 16 weeks. Other LNP preparations were stored at 4-8°C for 16 weeks, and the particle size, PDI, and cell transfection efficiency also maintained the initial state.

### Example 5: In vivo transfection experiment of lipid nanoparticle (LNP preparation)

LNP lipid nanoparticles loaded with Fluc-mRNA (CleanCap^{®} FLuc mRNA (5 moU) L-7202 0.1/1/5 mg), MC3 (CAS No.: 1224606-06-7, Xiamen Sinopeg Biotechnology Co., Ltd.), C12-200 (CAS number: 1220890-25-4, synthesized according to literature PNAS, 2010, Vol (107), 5, 1864-1869) was prepared respectively according to the method in Example 2. The specific parameters are shown in Table 3. DSPC was purchased from AVT (Shanghai) Pharmaceutical Technology Co., Ltd. DMG-PEG2000 was purchased from AVT (Shanghai) Pharmaceutical Technology Co., Ltd. 6 mice (Fhenotek Biotechnology (Shanghai) Co., Ltd.) were randomly selected, and the lipid nanoparticles were injected intravenously and intramuscularly (3 mice in each group) at a dose of 0.5 mg/kg. DPBS and Fluc-mRNA were used as controls. After 6 hours, 200 ul 10 mg/ml D-luciferin potassium salt was injected into each mouse through the tail vein. After 10 minutes, the mouse were placed under the *in vivo* imaging system, the total fluorescence intensity of each mouse were observed, and photographed for record. Tissues (heart, liver, spleen, lung, kidney, brain, pancreas, muscle) were dissected and separated, and placed in a plate for luminescent photography. After 24 hours, the mice were placed under the in vivo imaging system, the total fluorescence intensity of each mouse was observed, and photographed for recorded.

**Table 3: Preparation parameters of each LNP**

| Sample Number | Composition and Proportion (Molar Ratio) | Ionizable lipids/mRNA (mass ratio) |
|---|---|---|
| MC3 | MC3/Chol/DSPC/DMG-PEG2000 =50/38.5/10/1.5 | 12/1 |
| C12-200 | C12-200/Chol/DOPE/DSPE-PEG200 0 =35/46.5/16/2.5 | 10/1 |
| CX-A | Target-1/Chol/DOPE/DSPE-PEG200 0 =35/46.5/16/2.5 | 10/1 |
| CX-B | Target-1/Chol/DSPC/DMG-PEG2000 =35/46.5/16/2.5 | 10/1 |

| | | |
|---|---|---|
| Note: Target-1 is compound B-C14. | | |

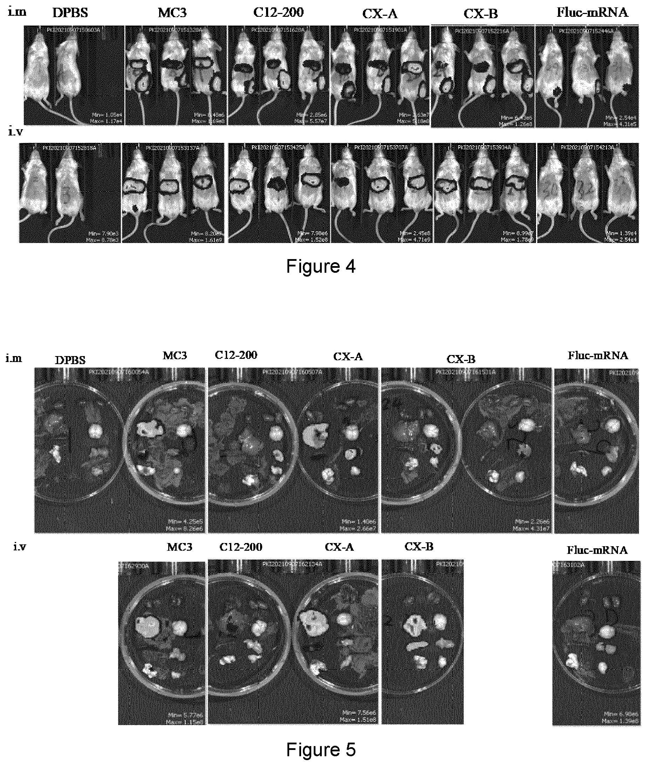

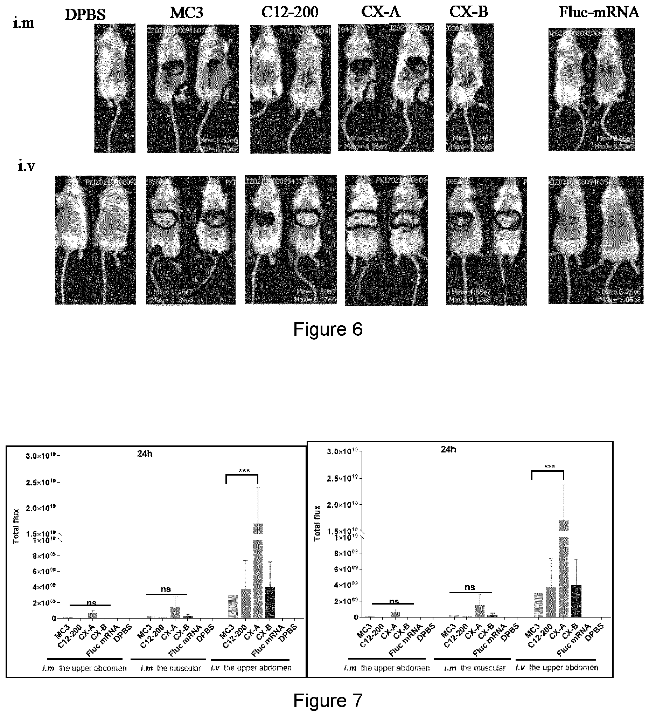

With intramuscular injection, the lipid nanoparticles will partially stay at the injection site, and the rest will mainly stay in the liver. By intravenous injection, all lipid nanoparticles were transferred to the chest and abdomen, mainly staying in the liver, followed by the spleen. As shown in Figure 4-7, compared with MC3 and C12-200, the fluorescence intensity of CX-A samples is higher. Especially after 24h, CX-A still maintained a high fluorescence intensity which was much higher than other groups. Other LNP formulations also maintained higher fluorescence intensities.

### Example 6: Safety experiment of ionizable lipid compound

The genotoxicity of ionizable lipid compounds was tested in accordance with the "Technical Guidelines for Drug Genotoxicity Research" in the "SFDA (State Food and Drug Administration) Guidelines". The results of bacterial reverse mutation test, mammalian erythrocyte micronucleus test and in vitro mammalian cell chromosome aberration test of the LNP preparation prepared in Example 2 were all negative.

The toxicity of empty-loaded LNP was tested according to the "Technical Guidelines for Single-dose Toxicity Studies of Drugs". The results showed that all the LNP preparations prepared in Example 2 did not cause acute toxicity.

The hemolysis of empty LNP was tested according to the 2020 edition of "Pharmacopoeia of the People's Republic of China" (Volume Four), General Principles, biological test method, 1148 hemolysis and coagulation test method. The results showed that no hemolysis and aggregation occurred for the LNP preparation prepared in Example 2.

## Claims

1. An ionizable lipid compound of formula I, stereoisomer, tautomer, pharmaceutically acceptable salt, prodrug, or solvate thereof: 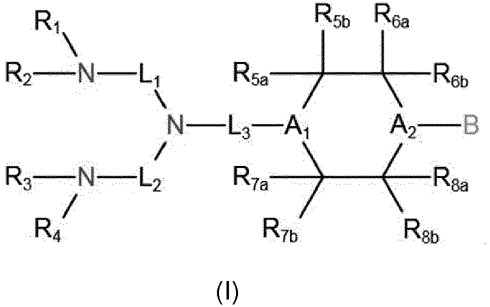 wherein,
A₁ and A₂ are each independently CH or N;
B is selected from -L₄-N (R₉R₁₀) or R₁₂;
L₁, L₂, L₃ and L₄ are each independently selected from optionally substituted alkylene, optionally substituted -[(CH₂)ₙO]ₘ-(CH2)_{b}-, optionally substituted alkenylene and optionally substituted alkynylene;
R₁, R₂, R₃, R₄, R₉, R₁₀ and R₁₂ are each independently H, halogen, optionally substituted alkyl, optionally substituted alkenyl, optionally substituted cycloalkyl, optionally substituted heterocyclyl, optionally substituted cycloalkenyl, optionally substituted cycloalkynyl, optionally substituted aryl, optionally substituted heteroaryl, optionally substituted alkoxy, optionally substituted alkoxycarbonyl, optionally substituted acyl, -NR'R", carboxy, nitro, cyano, or alkylsulfoxide;
R₅ₐ, R_{5b}, R₆ₐ, R_{6b}, R₇ₐ, R_{7b}, R₈ₐ and R_{8b} are each independently selected from H, optionally substituted alkyl, optionally substituted alkenyl, optionally substituted alkynyl, halogen, nitro, cyano, -NR'R" and carboxy;
n, m, and o are each independently an integer from 1 to 10; and
R' and R" are each independently selected from H or optionally substituted alkyl.

2. The ionizable lipid compound or stereoisomer, tautomer, pharmaceutically acceptable salt, prodrug or solvate thereof according to claim 1, wherein,
both A₁ and A₂ are N; and/or
L₁, L₂, L₃ and L₄ are each independently C₁₋₄ alkylene or -[(CH₂)ₙO]ₘ-(CH₂)ₒ- optionally substituted with 1-5 substituents selected from hydroxyl, -NR'R", C₁₋₄ alkoxy and halogen, wherein, R' and R" are each independently selected from H and C₁₋₄ alkyl; more preferably, L₁, L₂, L₃ and L₄ are each independently C₁₋₄alkylene or -[(CH₂)ₙO]ₘ-(CH₂)ₒ-; preferably, n, m and o are each independently an integer from 1 to 4; more preferably, n is 2, m is 1-4, o is 2; more preferably, L₁, L₂, L₃ and L₄ are all C₁₋₄ alkylene, or L₁, L₂ and L₄ are each independently -[(CH₂)ₙO]ₘ-(CH₂)ₒ-, preferably, L₁ , L₂ and L₄ are the same group, L₃ is C₁₋₄ alkylene; and/or
R₅ₐ, R_{5b}, R₆ₐ, R_{6b}, R₇ₐ, R_{7b}, R₈ₐ and R_{8b} are each independently selected from H and C₁₋₄ alkyl optionally substituted with 1-5 substituents selected from hydroxyl and halogen; preferably, R₅ₐ, R_{5b} , R₆ₐ, R_{6b}, R₇ₐ, R_{7b}, R₈ₐ and R_{8b} are all H.

3. The ionizable lipid compound or stereoisomer, tautomer, pharmaceutically acceptable salt, prodrug or solvate thereof according to any one of claims 1-2, wherein,
R₁, R₂, R₃ and R₄ are each independently C₁₋₂₄ alkyl substituted with hydroxyl and/or halogen, preferably are each independently C₈₋₂₄ alkyl substituted with hydroxyl and/or halogen; preferably, a hydrogen on the second carbon atom of the alkyl from the N atom connecting end is substituted by one hydroxyl; preferably R₁, R₂, R₃ and R₄ are each independently -CH₂CH(OH)CH₂Rₙ, wherein, the R₁₁ is a C₁₋₂₁ alkyl unsubstituted or halogen substituted, preferably is a C₁₋₂₁ alkyl unsubstituted or perfluorinated; preferably, R₁, R₂, R₃ and R₄ are the same group; and/or
R₉, R₁₀ and R₁₂ are each independently C₁₋₂₄ alkyl optionally substituted by hydroxyl and/or halogen, preferably each independently is C₈₋₂₄ alkyl optionally substituted by hydroxyl and/or halogen; preferably, a hydrogen on the second carbon atom of the alkyl from the N atom connecting end is substituted by one hydroxyl; preferably, R₉ and R₁₀ are each independently -CH₂CH(OH)CH₂R₁₁, wherein the R₁₁ is a C₁₋₂₁ alkyl unsubstituted or halogen substituted, preferably is a C₁₋₂₁ alkyl unsubstituted or perfluorinated; preferably, both R₉ and R₁₀ are the -CH₂CH(OH)CH₂R₁₁; preferably, R₁₂ is a C₁₋₁₀ alkyl optionally substituted by 1-5 hydroxyl, preferably, a hydrogen on the second carbon atom of the alkyl from the N atom connecting end is substituted by one hydroxyl;
more preferably, R₁, R₂, R₃, R₄, R₉ and R₁₀ are the same group.

4. The ionizable lipid compound or stereoisomer, tautomer, pharmaceutically acceptable salt, prodrug or solvate thereof according to claim 1, wherein,
both A₁ and A₂ are N;
R₁, R₂, R₃ and R₄ are each independently C₁₋₂₄ alkyl optionally substituted by hydroxyl and/or halogen; preferably, R₁, R₂, R₃ and R₄ are substituted by at least one hydroxyl; preferably, R₁, R₂, R₃ and R₄ are each independently -CH₂CH(OH)CH₂Rₙ, wherein, R₁₁ is a C₁₋₂₁ alkyl optionally substituted by halogen, preferably R₁₁ is an unsubstituted C₁₋₂₁ alkyl or perfluorinated C_{1 -21} alkyl;
R₅ₐ, R_{5b}, R₆ₐ, R_{6b}, R₇ₐ, R_{7b}, R₈ₐ and R_{8b} are all H;
B is -L₄-N(R₉R₁₀); wherein, L₁, L₂, L₃ and L₄ are all C₁₋₄ alkylene, preferably, L₁, L₂, L₃ and L₄ are the same alkylene; or L₁, L₂ and L₄ are each independently -[(CH₂)ₙO]ₘ-(CH₂)ₒ-, preferably, L₁, L₂ and L₄ are the same group, and L₃ is C₁₋₄ alkylene; R₉ and R₁₀ are each independently C₁₋₂₄ alkyl optionally substituted by hydroxyl and/or halogen; preferably, R₉ and R₁₀ are substituted by at least one hydroxyl; preferably, R₉ and R₁₀ are each independently -CH₂CH(OH)CH₂R₁₁, wherein, the R₁₁ is a C₁₋₂₁ alkyl optionally substituted by halogen, preferably, R₁₁ is an unsubstituted C₁₋₂₁ alkyl or a perfluorinated C₁₋₂₁ alkyl;
or B is R₁₂; wherein, R₁₂ is a C₁₋₁₀ alkyl optionally substituted by 1-5 hydroxyl, preferably, a hydrogen on the second carbon atom of the alkyl from the N atom connecting end is substituted by one hydroxyl; L₁ and L₂ are each independently -[(CH₂)ₙO]ₘ-(CH₂)_{b}- or C₁₋₄ alkylene, preferably, L₁ and L₂ are the same group; L₃ is C₁₋₄ alkylene;
n, m, and o are each independently an integer from 1 to 4.

5. The ionizable lipid compound or stereoisomer, tautomer, pharmaceutically acceptable salt, prodrug or solvate thereof according to claim 1, wherein, the compound of formula I has the following structure: 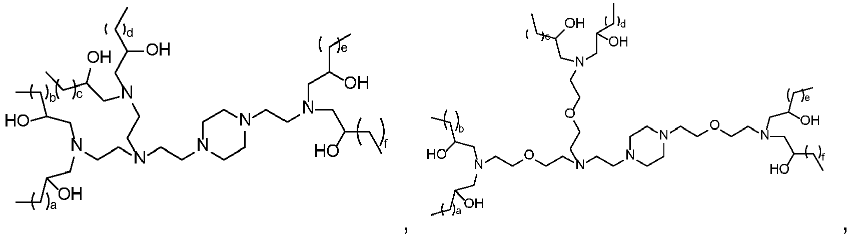 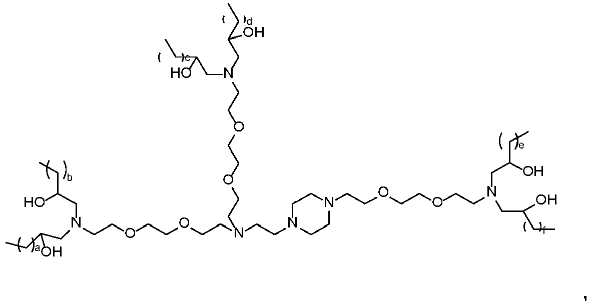 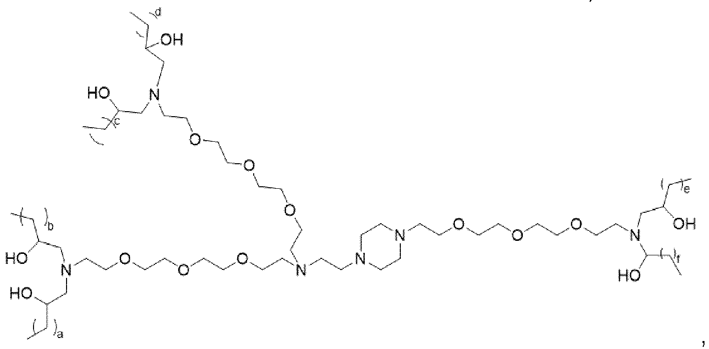 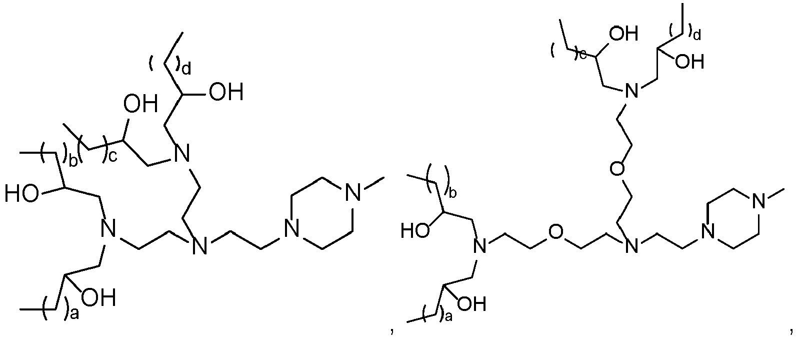 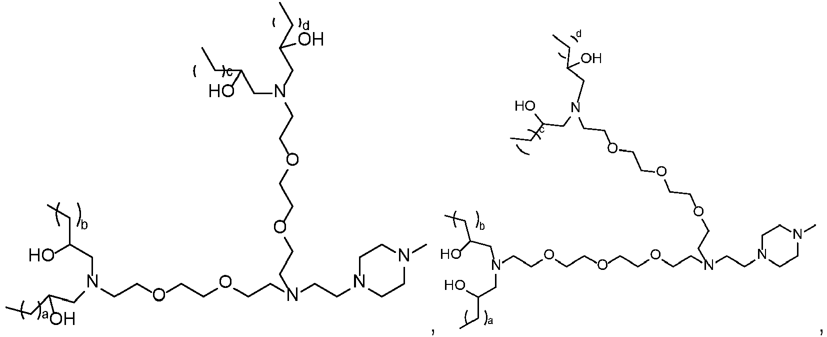 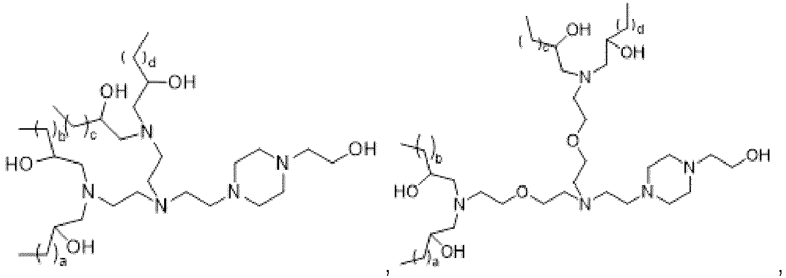 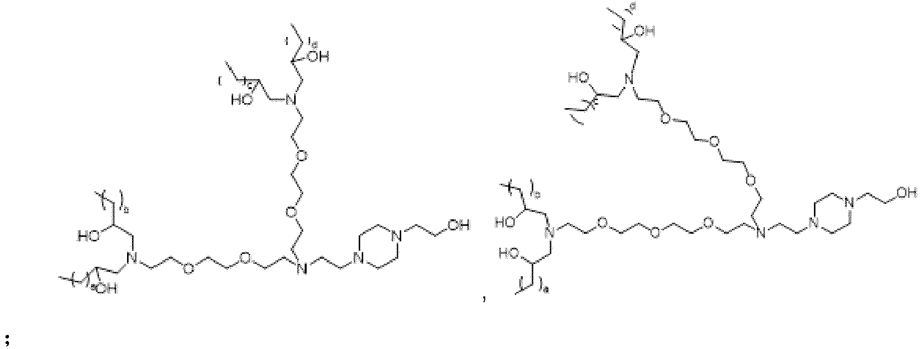 in each structure, a, b, c, d, e and f are each independently an integer from 0 to 20, preferably an integer from 8 to 16, preferably, a, b, c, d, e and f are the same integer selected from 0 to 20, preferably the same integer selected from 8 to 16.

6. The ionizable lipid compound or stereoisomer, tautomer, pharmaceutically acceptable salt, prodrug or solvate thereof according to claim 1, wherein, the compound of formula I is selected from compounds A-C12, A-C14, A-C16, A-C18, B-C12, B-C14, B-C16, B-C18, C-C12, C-C14, C-C16, C-C18, D-C12, D-C14, D-C16, D-C18, E-C12, E-C14, E-C16, E-C18, F-C12, F-C14, F-C16, F-C18, G-C12 , G-C14, G-C16, G-C18, H-C12, H-C14, H-C16, H-C18, I-C12, I-C14, I-C16, I-C18, J-C12, J-C14, J-C16, J-C18, K-C12, K-C14, K-C16, K-C18, L-C12, L-C14, L-C16 and L-C18.

7. A lipid nanoparticle, which comprises the ionizable lipid compound or stereoisomer, tautomer, pharmaceutically acceptable salt, prodrug or solvent thereof according to any one of claims 1-6; preferably, the lipid nanoparticle further comprises: one or more auxiliary lipid molecules, one or more cholesterol or cholesterol derivatives and/or one or more polymer-conjugated lipid molecular.

8. The lipid nanoparticle according to claim 7, wherein:
the auxiliary lipid molecule is a neutral lipid molecule, preferably selected from: DSPC, DPPC, DMPC, DOPC, POPC, DOPE and SM, more preferably DOPE or DSPC; and/or
the polymer of the polymer-conjugated lipid molecule is polyethylene glycol; preferably, the polymer-conjugated lipid molecule is selected from PEG-DAG, PEG-PE, PEG-S-DAG, PEG-DSPE, PEG-cer, DMG-PEG and PEG dialkoxypropyl carbamate, preferably PEG2000-DSPE or DMG-PEG2000.

9. The lipid nanoparticle according to any one of claims 7-8, wherein, in the lipid nanoparticle, the molar ratio of the ionizable lipid compound or stereoisomer, tautomer, pharmaceutically acceptable salt, prodrug or solvate thereof to the auxiliary lipid molecule, the cholesterol or cholesterol derivative, and the polymer-conjugated lipid molecule is (60 to 5):(60 to 5):(50 to 5):(10 to 1), preferably (40 to 10):(30 to 10):(50 to 20):(8 to 1).

10. The lipid nanoparticle according to any one of claims 7-9, wherein,
the lipid nanoparticles are used for expressing proteins encoded by mRNA, wherein, the proteins are employed for treatment, prevention, or improvement of physiological functions in organisms, including antigens and antibodies; or
the lipid nanoparticles are used to up-regulate endogenous protein expression by delivering a miRNA inhibitor targeting a specific miRNA or by delivering a panel of miRNAs that modulate a target mRNA or several mRNAs; or
the lipid nanoparticles are used to down-regulate protein levels and/or mRNA levels of target genes; or
the lipid nanoparticles are used to deliver mRNA and plasmids to express transgenes; or
the lipid nanoparticles are used to induce pharmacological effects resulting from protein expression.

11. The lipid nanoparticle according to any one of claims 7-10, wherein the lipid nanoparticle further comprises a therapeutic agent or active agent; preferably, the therapeutic agent or active agent is nucleic acid therapeutic agent or active agent; more preferably, the nucleic acid therapeutic agent or active agent is selected from: messenger RNA (mRNA), antisense oligonucleotide (ASO), small interfering RNA (siRNA), microRNA (miRNA), ribozymes and aptamers.

12. A composition comprising the ionizable lipid compound or stereoisomer, tautomer, pharmaceutically acceptable salt, prodrug or solvate thereof according to any one of claims 1-6;
preferably, the composition is a pharmaceutical composition, which comprises the ionizable lipid compound or stereoisomer, tautomer, pharmaceutically acceptable salt, prodrug or solvate thereof according to any one of claims 1-6, a therapeutic agent or active agent, and one or more auxiliary lipid molecules, one or more cholesterol or cholesterol derivatives and/or one or more polymer-conjugated lipid molecules.

13. The composition according to claim 12, wherein,
the auxiliary lipid molecule is a neutral lipid molecule, preferably selected from: DSPC, DPPC, DMPC, DOPC, POPC, DOPE and SM, more preferably DOPE; and/or
the polymer of the polymer-conjugated lipid molecule is polyethylene glycol; preferably, the polymer-conjugated lipid molecule is selected from PEG-DAG, PEG-PE, PEG-S-DAG, PEG-DSPE, PEG-cer and PEG dialkoxypropyl carbamate, preferably PEG2000-DSPE; and/or
the therapeutic agent or active agent is a nucleic acid therapeutic agent or active agent; preferably, the nucleic acid therapeutic agent or active agent is selected from: messenger RNA (mRNA), antisense oligonucleotide (ASO), small interfering RNA (siRNA), microRNA (miRNA), ribozymes and aptamers.

14. The composition according to claim 12 or 13, wherein the composition further comprises apolipoprotein; preferably, the apolipoprotein is selected from the group consisting of: ApoA-I, ApoA-II, ApoA-IV, ApoA-V and ApoE, and their active polymorphs, isoforms, variants and mutants and fragments or truncated forms thereof, preferably ApoE4.

15. Use of the ionizable lipid compound or stereoisomer, tautomer, pharmaceutically acceptable salt, prodrug or solvate thereof according to any one of claims 1-6 in manufacture of lipid nanoparticles or pharmaceutical compositions for treatment or prevention of disease or condition in a subject, or in manufacture of reagents for the delivery of therapeutic agents or active agents such as nucleic acid molecules.
